# EUROPEAN PATENT SPECIFICATION

(11) **EP 4 114 440 B1**
(45) Date of publication and mention of the grant of the patent: **25.06.2025**
(21) Application number: 21709019.0
(22) Date of filing: 05.03.2021
(51) Int. Cl.: A61K 38/20, A61K 9/00, A61P 25/28

(54) **LOW DOSE HUMAN INTERLEUKIN-2 FOR THE TREATMENT OF AMYOTROPHIC LATERAL SCLEROSIS**
INTERLEUKIN-2 IN NIEDRIGER DOSIS ZUR BEHANDLUNG DER AMYOTROPHEN LATERALSKLEROSE
TRAITEMENT DE LA SCLÉROSE LATÉRALE AMYOTROPHIQUE A BASE DE FAIBLES DOSES D' INTERLEUKINE-2

(30) Priority: 06.03.2020 EP 20305241
(43) Date of publication of application: 11.01.2023
(73) Proprietor: Centre Hospitalier Universitaire de Nimes, 30029 Nimes Cedex 9 (FR); Humanitas Mirasole SpA, 20100 Rozzano (MI) (IT); The University of Sussex, Brighton, East Sussex BN1 9RH (GB); King's College London, London WC2R 2LS (GB); Queen Mary University of London, London E1 4NS (GB); SORBONNE UNIVERSITE, 75006 Paris (FR); Assistance Publique - Hôpitaux de Paris, 75012 Paris (FR); The University of Sheffield, Sheffield S10 2TN (GB)
(72) Inventor: BENSIMON, Gilbert, 94800 VILLEJUIF (FR); LEIGH, Peter Nigel, ROBERTSBRIDGE TN32 5NB (GB); TREE, Timothy, LONDON SE22 8DE (GB); GARLANDA, Cecilia, 20148 MILANO (IT); LOCATI, Massimo, 20089 ROZZANO MI (IT); KIRBY, Janine, DONCASTER DN7 4BU (GB); SHAW, Pamela, HOPE VALLEY DERBYSHIRE S33 9JQ (GB); MALASPINA, Andrea, LONDON E1 4NS (GB)
(74) Representative: Cabinet Becker et Associés
(86) International application number: PCT/EP2021/055570
(87) International publication number: WO 2021/176044

(56) References cited:
- EP-A1- 1 939 216
- WO-A1-2012/123381
- US-A- 5 145 677
- GILBERT BENSIMON ET AL: "IMMUNO- MODULATION IN AMYOTROPHIC LATERAL SCLEROSIS A PHASE 11 STUDY OF SAFETY AND ACTIVITY OF LOW DOSE INTERLEUKIN-2 (IMODALS study) (S3.006)", INTERNET CITATION, 18 April 2017 (2017-04-18), XP009544532, ISSN: 0028-3878, Retrieved from the Internet <URL:https://n.neurology.org/content/88/16_Supplement/S3.006>
- SHEEAN RK ET AL., JAMA NEUROL, vol. 75, no. 6, 2018, pages 681 - 689
- THONHOFF JASON R ET AL: "Expanded autologous regulatory T-lymphocyte infusions in ALS: A phase I, first-in-human study", NEUROLOGY: NEUROIMMUNOLOGY & NEUROINFLAMMATION, AMERICAN ACADEMY OF NEUROLOGY, US, vol. 5, no. 4, 30 June 2018 (2018-06-30), pages e465 - 1, XP009521338, ISSN: 2332-7812, DOI: 10.1212/NXI.0000000000000465

## Description

### TECHNICAL FIELD OF THE INVENTION

The present invention is in the field of amyotrophic lateral sclerosis (ALS). It relates to human interleukin-2 (IL-2) for use in the treatment of amyotrophic lateral sclerosis in a human subject, wherein each dose of human IL-2 administered to said subject is between 0.1 x10⁶ to 3x10⁶ international units (IU). Human IL-2 is preferably administered as sub-cutaneous injections of 0.1 x10⁶ to 3x10⁶ IU of human IL-2. The treatment does not comprise the administration of regulatory T-cells to the subject, who is preferably also under riluzole treatment. The administered human IL-2 is not complexed with anti-hIL-2 antibodies and the treatment also does not comprise the administration of rapamycin or any other suppressive agent of effector T cells (Teffs) to the subject. Low dose IL2 treatment in subjects with ALS permits to (i) increase regulatory T-cell suppressive control over Teffs, (ii) decrease plasma CCL2 concentration, (iii) change polarization of blood macrophages from an M1 inflammatory phenotype to an anti-inflammatory M2 phenotype involved in tissue repair, and (iv) decrease overall ALS-related cytopathic activity.

### BACKGROUND ART

Amyotrophic Lateral Sclerosis (ALS) is a fatal neuromuscular disease, clinically characterized by relentlessly progressive weakness, muscle wasting and loss of motor function. Despite the introduction of riluzole two decades ago (Bensimon G et al N Engl J Med 1994; 330: 585-91;), subsequent trials have failed to deliver more effective disease-modifying remedies.

The many drug development failures in ALS are mainly related to the fact that ALS is a complex disease, for which there is no validated predictive animal model. In particular, while transgenic mutant SOD1 (mSOD1) mice are often used as an ALS animal model, all positive results obtained in this model have failed to translate into efficient remedies for human subjects with ALS (DiBernardo AB et al. Biochimica et Biophysica Acta 1762 (2006) 1139-1149; van den Berg LH et al. Neurology 2019;92:e1610-e1623). Neuro-inflammatory processes are prominent pathological features in subjects with ALS. Microglial cell activation is evidenced in the pathology of ALS at all disease stages (Troost D et al. Neuropathol Appl Neurobiol 1990; 16: 401-10; Kawamata T et al. Am J Pathol 1992; 140: 691; Engelhardt JI et al. Arch Neurol 1993; 50: 30-6; Thonhoff JR et al. Curr Opin Neurol 2018; 31: 635-9) and in the transgenic SOD1 ALS mouse (Engelhardt JI et al. Arch Neurol 1993; 50: 30-6; McGeer PL et al. Muscle Nerve 2002; 26: 459-70; Hall ED, Oostveen JA, Gurney ME. Glia 1998; 23: 249-56) in which expression of macrophage-typical cytokines precedes clinical symptoms (Alexianu ME et al. Neurology 2001; 57: 1282-9; Hensley K et al. J Neurochem 2002; 82: 365-74). Furthermore, biomarkers of neuro-inflammation are elevated in subjects with ALS and have been shown to correlate with disease severity and predict disease progression (Gille B et al. J Neurol Neurosurg Psychiatry. 2019 Dec;90(12):1338-1346; Tateishi T et al. J Neuroimmunol. 2010 May;222(1-2):76-81).

Although evidence of a neuro-inflammatory contribution to ALS pathogenesis is compelling (Evans MC et al. Mol Cell Neurosci 2013; 53: 34-41; Zhao W et al. J Neuroimmune Pharmacol Off J Soc Neurolmmune Pharmacol 2013; 8: 888-99), until now all therapeutic attempts to modify the neuro-inflammatory response in the ALS clinical context have failed (Appel SH et al. Arch Neurol 1988; 45: 381; Drachman DB et al. Ann Neurol 1994; 35: 142-50; Tan E et al. Arch Neurol 1994; 51: 194; Beghi E et al. Neurology 2000; 54: 469-469; Cudkowicz MEet al. Ann Neurol 2006; 60: 22-31; Gordon PH et al. Lancet Neurol 2007; 6: 1045-53 ; Stommel EW et al. Amyotroph Lateral Scler 2009; 10: 393-404). However, most of these trials have targeted non-specific suppression of neuro-inflammation. Such approaches have foremost a high risk to harm subjects with ALS, where toxicity may easily outweigh a beneficial drug effect.

In this context, new approaches reinforcing physiological tolerogenic dominance within the neuroimmuno-inflammatory system without suppressing all immunity are needed. CD4+FOXP3+ regulatory T cells (Tregs) physiologically regulate immune responses, contributing to the induction and maintenance of tolerance thus preventing the onset of autoimmune and inflammatory diseases (Sakaguchi S et al. Cell 2008; 133: 775-87). Previous studies have shown that in ALS subjects decreased levels of Tregs were correlated with increased disease severity and were predictive of disease progression and survival (Mantovani S et al. J Neuroimmunol 2009; 210: 73-9; Rentzos M et al. Acta Neurol Scand 2012; 125: 260-4; Henkel JS et al. EMBO Mol Med 2013; 5: 64-79). In addition to their decreased levels, Tregs of ALS subjects expressed lower levels of FOXP3 (Henkel JS et al. EMBO Mol Med 2013; 5: 64-79) and were shown to be dysfunctional, their dysfunction correlating with increased disease severity (Beers DR et al. JCI Insight. 2017;2(5):e89530; Thonhoff JR et al. Curr Opin Neurol 2018; 31: 635-9). Therefore, in ALS subjects, Tregs are not only reduced in numbers but also show significant dysfunction correlated to impaired FOXP3 expression level, and their dysfunction correlates to disease severity and progression, suggesting that Treg suppressive function might be a meaningful indicator of clinical status (Thonhoff JR et al. Curr Opin Neurol 2018; 31: 635-9).

Tregs are exclusively reliant on the cytokine Interleukin 2 (IL-2) for their generation, activation and survival (Malek TR, Bayer AL. Nat Rev Immunol 2004; 4: 665-74). Moreover, contrary to human effector T-cells (Teffs), human Tregs constitutively express high levels of CD25, forming a high-affinity receptor for IL-2 and thus respond to low concentrations of IL-2, insufficient to stimulate Teffs (Dupont G. et al. Cytokine. 2014 Sep;69(1):146-9). On this basis, low dose (ld) IL-2 administration has been tested and shown to induce the selective expansion of Tregs in mice and humans in Healthy Volunteers or type 1 diabetes contexts (Hartemann A et al. Lancet Diabetes Endocrinol 2013; 1: 295-305 Ito S et al. Mol Ther J Am Soc Gene Ther 2014. 22: 1388-95).

On this basis, use of ld-IL-2 has been proposed in the treatment of various auto-immune and inflammatory conditions (WO2012123381A1, WO2014023752A1, WO2016025385A1, WO2016164937A2) and several clinical trials exploring the therapeutic potential of ld-IL-2 in graft-versus-host disease (Koreth J et al. N Engl J Med 2011; 365: 2055-66), HCV-induced vasculitis (Saadoun D et al. N Engl J Med 2011; 365: 2067-77), type 1 diabetes (Hartemann A et al. Lancet Diabetes Endocrinol 2013; 1: 295-305), alopecia areata (Castela E et al. JAMA Dermatol 2014; 150: 748-51) have now been reported.

In the context of an ALS animal model, it has been shown that administration of a combination of immune complexes of murine Il-2 (complexes of IL2 and anti-IL-2 antibodies) and rapamycin to the transgenic SOD1 ALS mouse resulted in a significant slowing of disease progression, accompanied by an increase in survival time (Sheean RK et al. JAMA Neurol. 2018;75(6):681-689). However, as explained above, many positive results obtained in this model with new candidate drugs have failed to translate into efficient remedies for human ALS subjects (DiBernardo AB et al. Biochimica et Biophysica Acta 1762 (2006) 1139-1149; van den Berg LH et al. Neurology 2019;92:e1610-e1623), including vitamin E (Desnuelle C. et al, Amyotroph. Lateral Scler. Other Motor Neuron Disord. 2 (2001) 9-18), gabapentin (Miller R.G., et al, Neurology 56 (2001) 843-848), topiramate (Cudkowicz M., et al, Neurology 61 (2003) 456-464), celecoxib (Cudkowicz M., 15th International Symposium on ALS/MND, Philadelphia, 2004), and creatine (Shefner J. et al, Neurology 63 (2004) 1656-1661).

Moreover, it has been claimed that Tregs from ALS human subjects have impaired endogenous responsiveness to IL-2 (Thonhoff JR et al. Neurol Neuroimmunol Neuroinflammation 2018; 5: e465 ), potentially making them unresponsive to ld IL-2 treatment, which implies that the same strategy cannot be efficient in ALS subjects. In particular, based on a first pilot study in 5 human ALS subjects (unpublished data), Thonhoff et al indicated that ldIL-2 was not found to alter the clinical outcome or to increase endogenous Treg numbers (see Discussion §2 of Thonhoff JR et al. Neurol Neuroimmunol Neuroinflammation 2018; 5: e465). On this basis, an alternative autologous cell therapy treatment consisting of ex-vivo isolation of Tregs from the ALS subject, followed by ex-vivo expansion of the subject's Tregs using both IL-2 and rapamycin, and reinjection to the subject of its expanded Tregs, concomitantly with ldIL-2 administration, has been proposed and said to be efficient for reducing rate of functional decline in only 3 ALS subject (Thonhoff JR et al. Neurol Neuroimmunol Neuroinflammation 2018; 5: e465).

However, results observed in a non-blind, non-placebo-controlled trial on only 3 subjects are clearly not sufficient for showing efficiency of a treatment in ALS. Moreover, the need to isolate subjects' Tregs and expanding them in vitro before reinjecting them to the subjects would make such a treatment very expensive and impossible to implement in many hospitals. There is thus still a need for alternative ALS treatments, which would be efficient but also less expensive and accessible to most ALS subjects due to a much simpler protocol.

Bensimon et al, 18 April 2017 ("Immuno-modulation in amyotrophic lateral sclerosis: a phase II study of safety and activity of low dose interleukin-2 (Imodals study)" S3.006), is an abstract retrieved from Neurology ISSN: 0028-3878 that reports preliminary results of an increase of Tregs following administration of low dose IL-2 in patients with ALS.

### SUMMARY OF THE INVENTION

In the context of the present invention, the inventors surprisingly found that the mere injection of low dose human interleukin 2 (ldIL-2) is sufficient to induce a significant improvement not only in Treg numbers but also most importantly in their suppressive function, without the need for Treg isolation and ex vivo expansion (see Example 1). In addition, the inventors also found that ldIL-2 not only increased Tregs numbers and function, but also resulted in a significant decrease in the plasma concentration of the inflammatory chemokine CCL2 (see Example 1), a small chemokine belonging to C-C subfamily also known as MCP1, which signals through the chemokine receptor-2 (CCR2), driving circulating leucocytes towards sites of neuroinflammation. Importantly, CCL2 plasma or CSF levels was shown to correlate with ALS disease score (Nagata T, et al. Neurol Res. 2007 Dec;29(8):772-6), and foremost with survival of ALS subjects (Gille B et al. J Neurol Neurosurg Psychiatry. 2019 Dec;90(12):1338-1346), making it a useful biomarker of ALS disease activity. The inventors also found that ld IL-2 was further able to upregulate the expression of chemokines (CCL17, CCL18), indicating a change of the phenotype of macrophages from an M1 inflammatory polarization to an anti-inflammatory M2 phenotype involved in tissue repair (Mantovani A et al. J Pathol 2013; 229: 176-85; Mammana S et al. Int. J. Mol. Sci. 2018, 19(3), 831, see Example 1).

Finally, the inventors found that overall decrease in cytopathic processes resulted in decrease of plasma accumulation of NFL, a non-specific marker of axonal lesioning. Therefore, contrary to the conclusions of Thonhoff et al, which were based on a very low (only 5 subjects) and probably unrepresentative number of subjects, the inventors showed in a three-arm, randomized (1:1:1), double-blind, single-center study of 2 doses of ld-IL-2 in parallel versus placebo clinical trial including 36 subjects (12 in each of the 3 treatment arms), that ldIL-2 injection to ALS subjects is (i) safe and well tolerated, and is able both to (ii) upregulate Tregs numbers and suppressive function over Teffs, (iii) downregulate inflammatory markers of disease progression (CCL2), (iv) shift monocyte polarization from M1 pro-inflammatory phenotype to M2 anti-inflammatory and tissue repair phenotype, and (v) decrease overall ALS-related cytopathic activity as evidenced by plasma NFL response to treatment, indicative of decreased axonal lesioning.

The invention is as defined in the appended claims.

In a first aspect, the present invention thus relates to human interleukin-2 (IL-2) for use in the treatment of amyotrophic lateral sclerosis in a human subject, wherein each dose of human IL-2 administered to said subject is between 0.1 x10⁶ to 3x10⁶ international units (IU) and said treatment does not comprise the administration of regulatory T-cells to the subject and does not comprise the administration of rapamycin or any other suppressive agent of effector T cells (Teffs) to the subject,
wherein the human IL-2 administered to the patient is not complexed with anti-human IL-2 antibodies.

### DESCRIPTION OF THE FIGURES

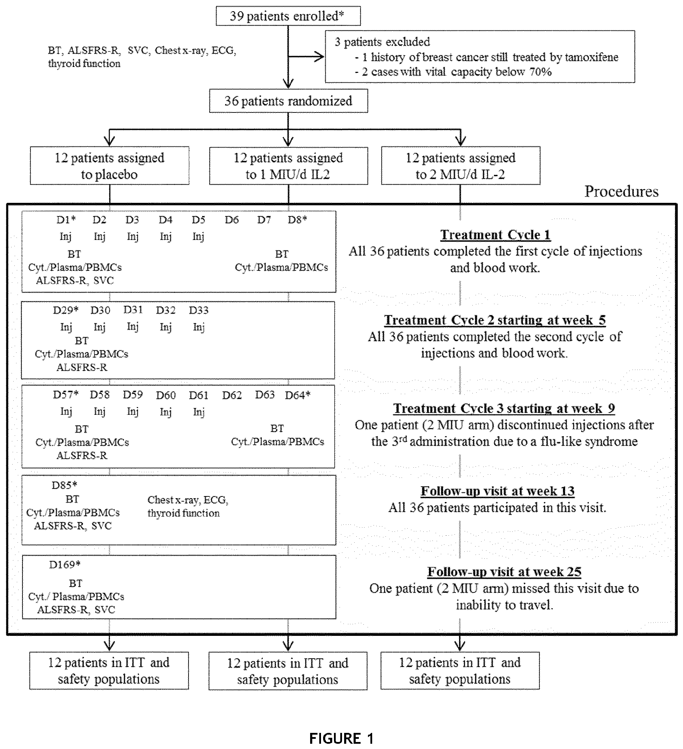

**Figure 1****:** Trial profile. ALSFRS-R: Amyotrophic Lateral Sclerosis Functional Rating Score - Revised; BT: Routine blood tests; Cyt: Fresh Blood Cytometry; SVC: slow vital capacity; PBMCs: Peripheral Blood Mononuclear Cells; Inj: sub-cutaneous injection; D: day; ECG: electrocardiogram; ITT: intention-to-treat. *Time frames corresponding to at-hospital visits.

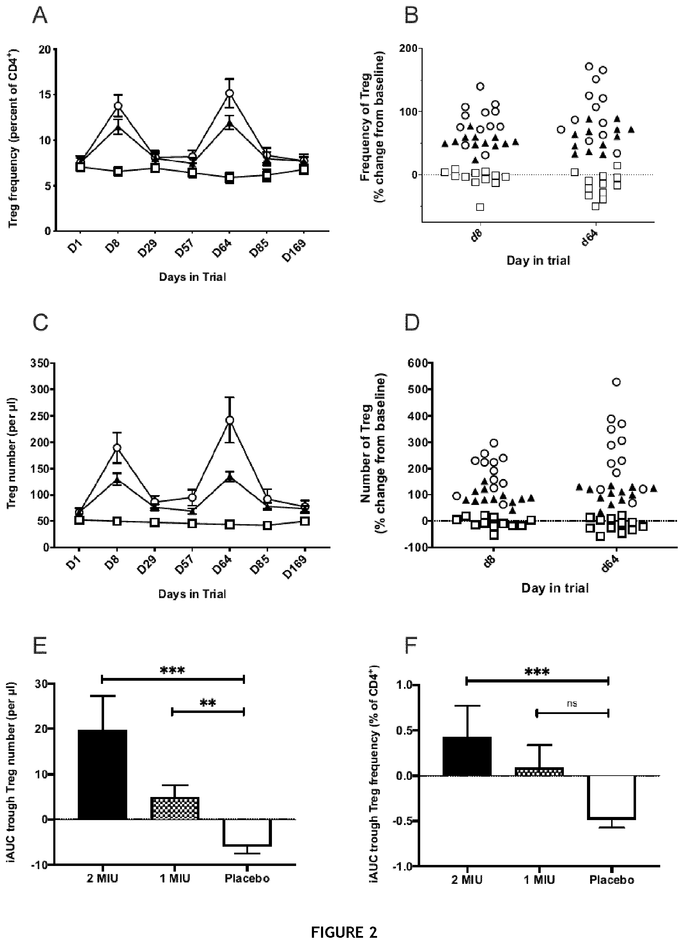

**Figure 2****.** Effect of IL-2 treatment on Treg number and frequency. A-D Variation in frequency (A-B) and absolute number (C-D) of Tregs throughout the study for all three arms (open squares, placebo; solid triangles, 1 MIU of IL2; open circles, 2 MIU of IL2). A and C Data points indicate mean values and error bars their associated SEMs. B and D Change in the number and frequency of Tregs between baseline and the three days after the final injection of one treatment cycle (d8) or 3 treatment cycles (d64). Data points represent the per-patient change in Treg frequency (B) and number (D). E-F iAUC of trough levels of Tregs during the study. Data points indicate mean values and error bars their associated SEMs for Treg number (E) and frequency (F).

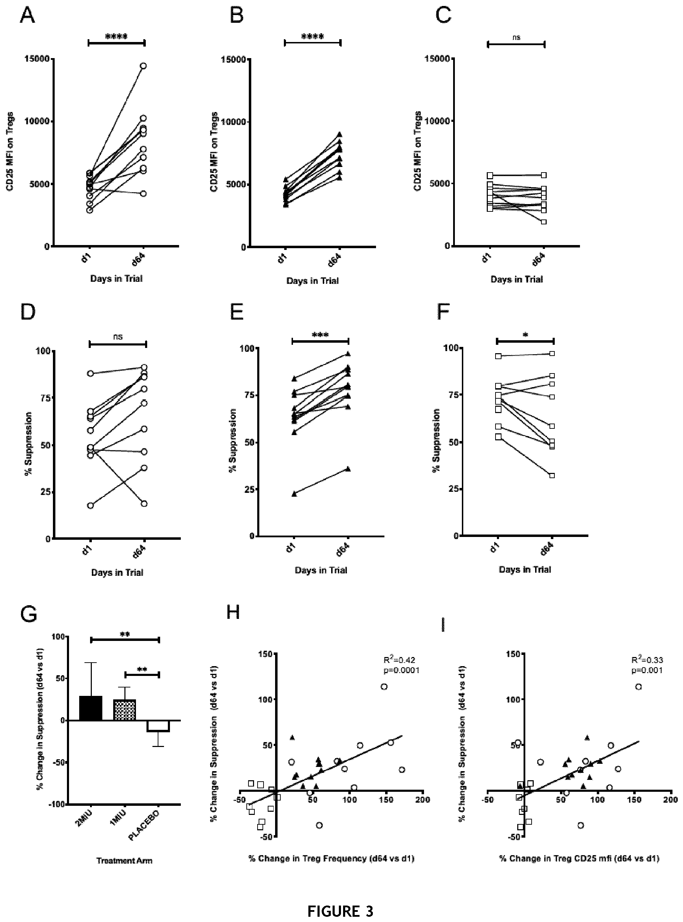

**Figure 3****.** Effect of IL-2 treatment on Treg frequency and effector T cell phenotype measured at baseline (d1) and 3 days after completion of 3 treatment cycles (d64). A-C frequency of Tregs isolated from cryopreserved PBMC for use in suppression assays in individuals treated with (A) 2MIU, (B) 1MIU and (C) placebo. D-F CD25 expression on effector T cells (D) 2MIU, (E) 1MIU and (F) placebo. G-I Proliferation of effector T cells in the absence of Tregs in individuals treated with (G) 2MIU, (H) 1MIU and (I) placebo.

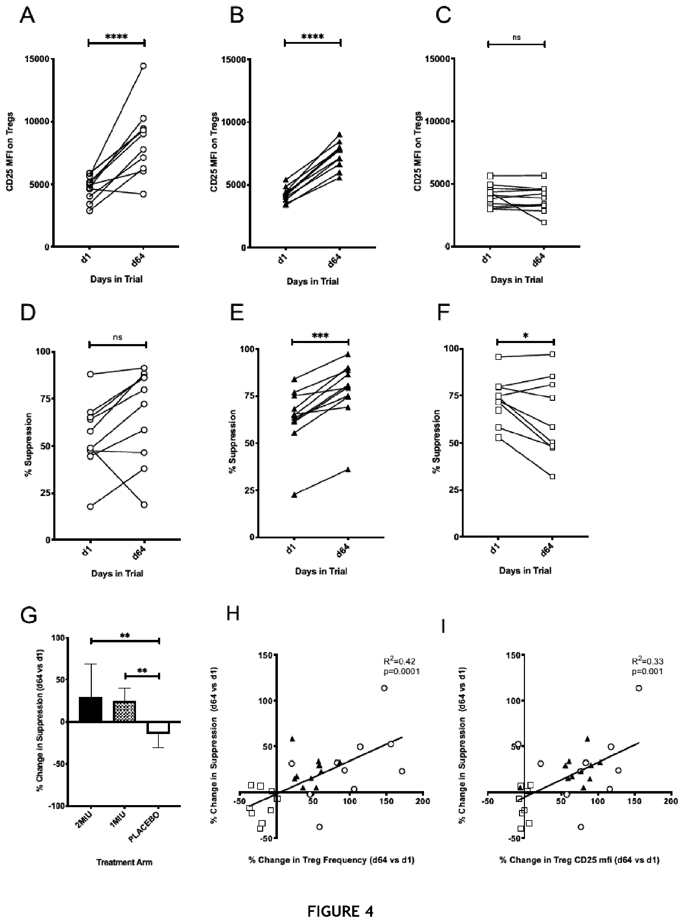

**Figure 4****.** Effect of IL-2 treatment on Treg phenotype and suppressive function. A-C CD25 expression on Tregs at baseline (d1) and 3 days after completion of 3 treatment cycles (d64) in all three study groups: (A) 2MIU, (B) 1MIU and (C) placebo. D-F Autologous suppressive function of Tregs measured by in vitro co-culture assay measured at baseline (d1) and 3 days after completion of 3 treatment cycles (d64) in individuals treated with (D) 2MIU, (E) 1MIU and (F) placebo. (G) Change in suppressive function of Tregs following 3 cycles of treatment relative to baseline levels in all three groups. Bars represent mean values and error bars their associated SEMs. (H-I) Relationship between the relative change in Treg frequency (H) and Treg CD35 mfi (I) measured by clinical cytometry (x-axis) and Treg suppressive function (Y axis) following 3 cycles of treatment (values at d64 vs d1). Open squares denote individuals receiving placebo, solid triangles 1 MIU and open circles 2 MIU of IL2.

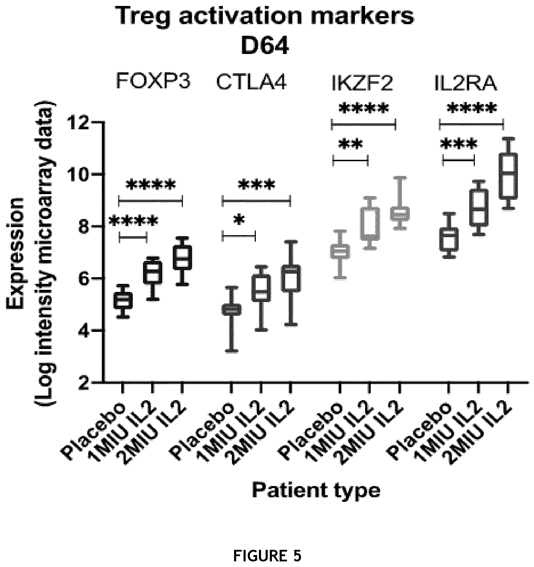

**Figure 5****.** Transcriptomic analysis of Treg activation markers FOXP3, CTLA4, IKZF2 and IL2RA (CD25) at D64. This plot shows increases in gene expression of the Treg activation markers FOXP3, CTLA4, IKZF2 and IL2RA in patients receiving 1MIU and 2MIU ldIL-2, compared to placebo. Each box represents the distribution of the expression values among each treatment group (maximum, minimum and median of the distributions are reported in each box and can be visualized as 3 horizontal lines). Multiple t-tests were performed to identify significant differences among sample groups. Key : *= p-value<0.05, **= p-value<0.01, ***= p-value<0.001, ****= p-value<0.0001.

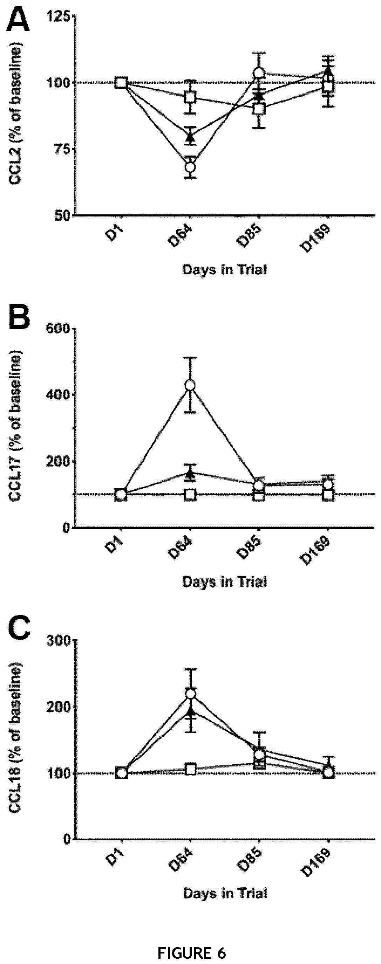

**Figure 6****.** Effect of IL-2 treatment on plasma cytokine concentrations. A-C Variation in plasma cytokine levels throughout the study for CCL2 (A), CCL17 (B) and CCL18 (C). Concentrations are expressed as a percentage of baseline value for each individual and points indicate mean values and error bars their associated SEMs. Open squares denote individuals receiving placebo, solid triangles 1 MIU and open circles 2 MIU of IL2.

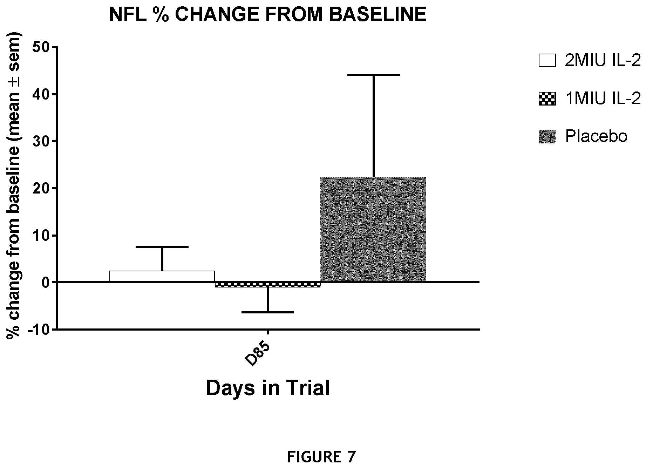

**Figure 7****.** Percentage of change from baseline for plasma NFL levels at D85 by treatment. bars are mean ± sem of percent change from baseline. solid grey= placebo; doted= 1MIU IL-2; white= 2MIU IL-2.

### DETAILED DESCRIPTION OF THE INVENTION

In the context of the present invention, the inventors surprisingly found that the mere injection of low dose human interleukin 2 (ldIL-2) is sufficient to induce a significant improvement not only in Treg numbers but also most importantly in their suppressive function in ALS subjects, without the need for ex vivo Tregs selection and expansion. In addition, the inventors also found that ldIL-2 not only increased Tregs numbers and function, but also resulted in a significant decrease in the plasma concentration of the inflammatory chemokine CCL2, a small chemokine belonging to C-C subfamily also known as MCP1, which signals through the chemokine receptor-2 (CCR2), driving circulating leucocytes towards sites of neuroinflammation and which has been shown to correlate with AlS disease score (Nagata T, et al. Neurol Res. 2007 Dec;29(8):772-6) and with survival of ALS subjects (Gille B et al. J Neurol Neurosurg Psychiatry. 2019 Dec;90(12):1338-1346), making it a useful biomarker of ALS disease activity. The inventors also found that ldIL-2 was further able to upregulate the expression of chemokines (CCL17, CCL18), indicating a change of the phenotype of macrophages from an M1 inflammatory polarization to an anti-inflammatory M2 phenotype involved in tissue repair (Mantovani A et al. J Pathol 2013; 229: 176-85; Mammana S et al. Int. J. Mol. Sci. 2018, 19(3), 831). Furthermore, all these changes are paralleled in the treated groups with a lasting arrest of NFL plasma increase contrary to what is observed in the placebo group, indicating a lasting positive effect on overall cytopathic ALS-related activity. Therefore, contrary to the conclusions of Thonhoff et al, which were based on a very low (only 5 subjects) and probably unrepresentative number of patients, the inventors showed in a three-arm, randomized (1:1:1), double-blind, single-center study of 2 doses of ld-IL-2 in parallel versus placebo clinical trial including 36 ALS subjects (12 in each of the 3 treatment arms) that ldIL-2 injection to ALS s is safe and well tolerated, and is able to (i) upregulate Tregs numbers and function (ii) shift monocyte polarization towards an anti-inflammatory phenotype, and (iii) downregulate markers of disease progression (CCL2, NFL).

### Use of low dose human IL-2 in the treatment of amyotrophic lateral sclerosis in a human subject

The present invention thus relates to human interleukin-2 (IL-2) for use in the treatment of amyotrophic lateral sclerosis in a human subject, wherein each dose of human IL-2 administered to said subject is between 0.1 x10⁶ to 3x10⁶ international units (IU) and said treatment does not comprise the administration of regulatory T cells to the subject.

It is herein described the use of human interleukin-2 (IL-2) for the manufacture of a drug for use in the treatment of amyotrophic lateral sclerosis in a human subject, wherein each dose of human IL-2 administered to said subject during said treatment is between 0.1 x10⁶ to 3x10⁶ international units (IU) and said treatment does not comprise the administration of regulatory T cells to the subject.

It is also described the use of human interleukin-2 (IL-2) in the treatment of amyotrophic lateral sclerosis in a human subject, wherein each dose of human IL-2 administered to said subject is between 0.1 x10⁶ to 3x10⁶ international units (IU) and said treatment does not comprise the administration of regulatory T cells to the subject.

The present invention also relates to a pharmaceutical composition comprising human interleukin-2 (IL-2) for use in the treatment of amyotrophic lateral sclerosis in a human subject, wherein each dose of human IL-2 administered to said subject is between 0.1 x10⁶ to 3x10⁶ international units (IU) and said treatment does not comprise the administration of regulatory T cells to the subject.

It is also described a method for treating amyotrophic lateral sclerosis in a human subject in need thereof, comprising administering to said human subject human interleukin-2 (IL-2), wherein each dose of human IL-2 administered to said subject is between 0.1 x10⁶ to 3x10⁶ international units (IU) and said treatment does not comprise the administration of regulatory T-cells to the subject.

### Human IL-2

The treatment according to the present disclosure relies on the administration of low doses of human IL-2 to the human ALS subject.

In the present description, ***"human interleukin 2*"** or ***"human IL-2"*** designates any source of human IL-2, including native human IL-2 or human IL-2 obtained by recombinant or synthetic techniques, including recombinant IL-2 polypeptides produced by microbial hosts. Nucleotide and amino acid sequences of native human IL-2 are disclosed, for instance, in the description of human IL-2 gene in Pubmed Entrez Gene reference 3558. A reference sequence for native human IL-2 protein may be found in NCBI Reference Sequence NP_000577.2 (version updated on January 5, 2020), while a reference sequence for native human IL-2 mRNA may be found in NCBI Reference Sequence NM_000586.4 (version updated on January 5, 2020).

Human IL-2 may consist of or comprise the native human IL-2 polypeptide sequence, or can be an active variant of the native human IL-2 polypeptide. Preferably recombinant human IL-2 is used, particularly recombinant human IL-2 produced by microbial hosts. Active variants of IL-2 have been disclosed in the literature. Variants of the native IL-2 can be fragments, analogues, and derivatives thereof. By "fragment" is intended a polypeptide comprising only a part of the intact polypeptide sequence. An "analogue" designates a polypeptide comprising the native polypeptide sequence with one or more amino acid substitutions, insertions, or deletions. Muteins and pseudopeptides are specific examples of analogues. "Derivatives" include any modified native IL-2 polypeptide or fragment or analogue thereof, such as glycosylated, phosphorylated, fused to another polypeptide or molecule, polymerized, etc., or through chemical or enzymatic modification or addition to improve the properties of IL-2 (e.g., stability, specificity, etc.). Active variants of native human IL-2 polypeptide generally have at least 75%, preferably at least 85%, more preferably at least 90% amino acid sequence identity to the amino acid sequence of native human IL-2 polypeptide. Methods for determining whether a variant IL-2 polypeptide is active are available in the art, examples of IL-2 variants being disclosed, for instance, in EP109748, EP136489, US4,752,585; EP200280, or EP118617. An active variant is, most preferably, a variant that activates Tregs. Preferably, recombinant human IL-2, i.e., human IL-2 that has been prepared by recombinant DNA techniques, is used. The host organism used to express a recombinant DNA encoding human IL-2 may be prokaryotic (a bacterium such as E. coli) or eukaryotic (e.g., a yeast, fungus, plant or mammalian cell). Processes for producing recombinant IL-2 have been described e.g., in US4,656,132; US4,748,234; US4,530,787; or US4,748,234. Human IL-2 for use in the present invention shall be in in pharmaceutically acceptable form, and notably in essentially pure form, e.g., at a purity of 95% or more, further preferably 96, 97, 98 or 99% pure.

Human IL-2 is commercially available, including for pharmaceutical uses, and has been authorized for use in human subjects. For instance, aldesleukin (trademark name Proleukin^{®}) is an analog of the human interleukin-2 gene produced by recombinant DNA technology using a genetically engineered E. coli strain, which has been approved by the FDA in the treatment of cancers. Aldesleukin differs from native human interleukin-2 in the following ways:
a) aldesleukin is not glycosylated because it is derived from E. coli;
b) the molecule has no N-terminal alanine; the codon for this amino acid was deleted during the genetic engineering procedure;
c) the molecule has serine substituted for cysteine at amino acid position 125.

Aldesleukin will preferably be used in the invention.

However, other recombinant human IL-2 are available, such as:
- Roncoleukin^{®}, a medicinal form of recombinant human IL-2, isolated and purified from cells of the yeast Saccharomyces cerevisiae containing the gene of human IL-2;
- Interking, a recombinant IL-2 with a serine at residue 125, sold by Shenzhen Neptunus;
- Albuleukin, a recombinant human interleukin-2 (rIL-2) genetically fused to recombinant human serum albumin (rHSA).

### Human IL-2 dose and administration schemes and routes

As explained above, human Tregs constitutively express high levels of CD25, forming a high-affinity receptor for IL-2 absent on resting Teffs, and thus respond to low concentrations of IL-2, insufficient to stimulate Teffs. Since the treatment as described herein is intended to expand the number and function of Tregs but not to expand or stimulate Teffs, each dose of human IL-2 administered to the ALS subject is kept low, between 0.1 x10⁶ to 3x10⁶ international units (IU). In addition, in the context of type 1 diabetes, it has been shown that doses up to 3x10⁶ IU were safe, although more non-serious adverse events occurred at the highest dose of 3x10⁶ IU (Hartemann A et al. Lancet Diabetes Endocrinol 2013; 1: 295-305). Each administered human IL-2 dose should also be kept to at most 3x10⁶ IU in order to limit possible toxicity.

As explained above, Tregs are exclusively reliant on IL-2 for their generation, activation and survival (Malek TR, Bayer AL. Nat Rev Immunol 2004; 4: 665-74). Moreover, the half-like of aldesleukin administered to human patients is typically about 2-3 hours (see e.g. Proleukin^{®} label). Therefore, in order to obtain sustained expansion of Treg numbers and immunosuppressive function, human IL-2 is typically administered repeatedly to the ALS subjects.

In a preferred embodiment, human IL-2 is administered as repeated, preferably sub-cutaneous, injections of 0.1 x10⁶ to 3x10⁶ IU of human IL-2, preferably 0.2 x10⁶ to 3x10⁶ IU of human IL-2, 0.3 x10⁶ to 3x10⁶ IU of human IL-2, 0.4 x10⁶ to 3x10⁶ IU of human IL-2, more preferably 0.5 x10⁶ to 3x10⁶ IU of human IL-2, 0.6 x10⁶ to 3x10⁶ IU of human IL-2, 0.7 x10⁶ to 3x10⁶ IU of human IL-2, 0.8 x10⁶ to 3x10⁶ IU of human IL-2, 0.9 x10⁶ to 3x10⁶ IU of human IL-2, 1 x10⁶ to 3x10⁶ IU of human IL-2, 1 x10⁶ to 2x10⁶ IU of human IL-2, in particular 0.5 x10⁶ IU of human IL-2, 1x10⁶ IU of human IL-2, or 2x10⁶ IU of human IL-2.

Injections may be performed according to various administration schemes, including:
- Administrating schemes based on repeated cycles of once-daily or several daily injections of human IL-2, separated by periods without human IL-2 injections; and
- Continuous metronomic administration, i.e. once-daily or several daily injections of human IL-2.

In the context of IMODALS clinical trial (see Example 1), it has been shown that, contrary to what Thonhoff et al had suggested (Thonhoff JR et al. Neurol Neuroimmunol Neuroinflammation 2018; 5: e465), a first 5 days cycle starting at D1 (cycle 1) of 1x10⁶ or 2x10⁶ IU/day human IL-2 administered once daily subcutaneously was sufficient to significantly increase Tregs numbers and function up to D29 (see **Figure 2****).** Moreover, further identical 5 days cycles at week 5 (cycle 2, starting on D29) and week 9 (cycle 3, starting on D57) have been administered (see **Figure 1****),** and the peak of Tregs numbers and frequencies during cycle 3 was higher than that observed during cycle 1, suggesting that successive treatment cycles have residual effects that might be cumulative. This suggestion is further supported by significantly higher iAUC trough T_{reg} levels (measuring the residual Treg change before beginning a new cycle) in IL2 arms as compared to placebo **(****Figures 2E-F****, Tables 3 and 4** above).

Therefore, a cycle of 3 to 7 consecutive days of once-daily sub-cutaneous injection of 0.1 x10⁶ to 3x10⁶ IU of human IL-2, preferably 0.2 x10⁶ to 3x10⁶ IU of human IL-2, 0.3 x10⁶ to 3x10⁶ IU of human IL-2, 0.4 x10⁶ to 3x10⁶ IU of human IL-2, more preferably 0.5 x10⁶ to 3x10⁶ IU of human IL-2, 0.6 x10⁶ to 3x10⁶ IU of human IL-2, 0.7 x10⁶ to 3x10⁶ IU of human IL-2, 0.8 x10⁶ to 3x10⁶ IU of human IL-2, 0.9 x10⁶ to 3x10⁶ IU of human IL-2, 1x10⁶ to 3x10⁶ IU human IL-2, 0.5 x10⁶ to 2x10⁶ IU of human IL-2, 1 x10⁶ to 2x10⁶ IU of human IL-2, in particular 0.5 x10⁶ IU of human IL-2, 1x10⁶ IU of human IL-2, or 2x10⁶ IU of human IL-2, is expected to significantly increase Tregs numbers during the cycle and at least up to 3 further weeks.

In the therapeutic uses or methods according to the invention, several cycles of 3 to 7 consecutive days of once-daily sub-cutaneous injection of 0.1 x10⁶ to 3x10⁶ IU of human IL-2, preferably 0.2 x10⁶ to 3x10⁶ IU of human IL-2, 0.3 x10⁶ to 3x10⁶ IU of human IL-2, 0.4 x10⁶ to 3x10⁶ IU of human IL-2, more preferably 0.5 x10⁶ to 3x10⁶ IU of human IL-2, 0.6 x10⁶ to 3x10⁶ IU of human IL-2, 0.7 x10⁶ to 3x10⁶ IU of human IL-2, 0.8 x10⁶ to 3x10⁶ IU of human IL-2, 0.9 x10⁶ to 3x10⁶ IU of human IL-2, 1x10⁶ to 3x10⁶ IU human IL-2, 0.5 x10⁶ to 2x10⁶ IU of human IL-2, 1 x10⁶ to 2x10⁶ IU of human IL-2, in particular 0.5 x10⁶ IU of human IL-2, 1x10⁶ IU of human IL-2, or 2x10⁶ IU of human IL-2, are thus preferably administered to the subject. More preferably, each cycle consists of 5 consecutive days of once-daily sub-cutaneous injection of 0.1 x10⁶ to 3x10⁶ IU of human IL-2, preferably 0.2 x10⁶ to 3x10⁶ IU of human IL-2, 0.3 x10⁶ to 3x10⁶ IU of human IL-2, 0.4 x10⁶ to 3x10⁶ IU of human IL-2, more preferably 0.5 x10⁶ to 3x10⁶ IU of human IL-2, 0.6 x10⁶ to 3x10⁶ IU of human IL-2, 0.7 x10⁶ to 3x10⁶ IU of human IL-2, 0.8 x10⁶ to 3x10⁶ IU of human IL-2, 0.9 x10⁶ to 3x10⁶ IU of human IL-2, 0.5 x10⁶ to 2x10⁶ IU of human IL-2, 1 x10⁶ to 2x10⁶ IU of human IL-2, in particular 0.5 x10⁶ IU of human IL-2, 1 x10⁶ to 3 x10⁶ IU IL-2, or 2x10⁶ IU human IL-2.

Since each cycle is expected to significantly increase Tregs numbers and function during the cycle and at least about 3 further weeks, the cycles are preferably administered every 2 to 6 weeks, preferably every 2 to 5 weeks, more preferably every 2 to 4 weeks, in particular every 2, 3 or 4 weeks.

However, while the above administrations schemes are preferred, other administration schemes able to significantly increase Tregs numbers and function without unacceptable toxicity may be defined by those skilled in the art, based on their knowledge of low dose IL-2 administration in humans in other contexts.

Indeed, while there were teachings in the art suggesting that ALS subjects had dysfunctional Tregs (Beers DR et al. JCI Insight. 2017;2(5):e89530; Thonhoff JR et al. Curr Opin Neurol 2018; 31: 635-9) and did not respond to low dose IL-2 administration (Thonhoff JR et al. Neurol Neuroimmunol Neuroinflammation 2018; 5: e465), contrary to other human subjects, it has now been shown in a three-arm, randomized (1:1:1), double-blind, single-centre study of 2 doses of ld-IL-2 in parallel versus placebo that ALS subjects are in fact able to favorably respond to ld-IL-2, resulting in a significant increase in Tregs numbers and function, and foremost decrease in CCL2 plasma concentration, a marker of ALS disease activity. Based on this unexpected finding of the inventors, knowledge of the effects of low dose IL-2 in other human subjects may now be used to design other suitable administration schemes.

For instance, while subcutaneous administration has been used in IMODALS clinical trial, it has been shown in human cancer subjects that IL-2 administered intravenously resulted in significant increase in Tregs numbers (Ahmadzadeh M, Rosenberg SA. Blood 2006;107:2409-14). Therefore, in the therapeutic uses described herein, human IL-2 is preferably administered via subcutaneous or intravenous route. Since subcutaneous is easier and better tolerated and shown to be efficient in the IMODALS clinical trial, subcutaneous route is nevertheless preferred.

With respect to the dose, while each single dose should not be higher than 3x10⁶ IU in order to limit possible toxicity, results obtained in IMODALS clinical trial showed that the effect of low dose human IL-2 on Tregs numbers and function in ALS subjects was dose-dependent, the highest effects being obtained with the highest dose of 2x10⁶ IU human IL-2 once daily during each of the 5 days cycles. Therefore, when using an administration scheme comprising repeated and separated cycles of low dose human IL-2 administration, during a cycle, a daily dose of 1x10⁶ IU to 2x10⁶ IU, preferably 2x10⁶ IU, is preferred. The daily dose may however be administered either in a single daily administration or in several separated lower doses. For instance, in order to reach a daily dose of 2x10⁶ IU, a single dose of 2x10⁶ IU may be administered once daily, or this daily dose of 2x10⁶ IU may be split into 2 or more lower doses, such as 2 doses of 1x10⁶ IU (for instance one in the morning and the other in the evening), 3 doses of 0.67x10⁶ IU (for instance one in the morning, one in the middle of the day and the 3^{rd} in the evening), or even 4 doses of 0.5x10⁶ IU. Such splitting of the daily dose may in particular be used in ALS subjects suffering from adverse events when administered a single daily dose of 2 to 3x10⁶ IU.

In addition, while the administration scheme used in IMODALS clinical trial is based on 5 days cycles every 4 weeks, other administration schemes may be contemplated. For instance, alternative administration schemes may be based on:
- longer cycles with a longer period between 2 cycles;
- shorter cycles with a shorter period between 2 cycles;
- continuous administration of low dose human IL-2 (no cycles).

For instance, when using administration cycles, while each cycle may notably vary between 3 and 7 days, shorter (such as 2 days) or longer (such as 8, 9, 10, 11, 12, 13, 14 days, or even 3 or 4 weeks) cycles may be used. When using shorter cycles (such as 2 days), the cycles will preferably be repeated more often than the every 4 weeks schedule used in IMODALS, such as every 3 weeks, every 2 weeks, every 10 days, or every week.

When using longer cycles (such as 8, 9, 10, 11, 12, 13, 14 days, or even 3 or 4 weeks), the cycles will preferably be repeated as often or a little bit less often than the every 4 weeks schedule used in IMODALS, such as every 4 weeks, every 5 weeks, or every 6 weeks. However, due to the relatively short duration of the effects of human IL-2, the period between cycles should not be too much increased.

Continuous- metronomic -administration of low dose human IL-2 (no cycles) may also be contemplated. While administration schemes based on repeated cycles of low dose human IL-2 have been used in IMODALS and in other autoimmune diseases based on previous knowledge derived from cancer therapy and for commodity for the subject, continuous - metronomic- administration of low dose human IL-2 may still be considered, in particular if a pump permitting continuous administration of low dose human IL-2 (similar to those used for delivering insulin to diabetic subjects) is used. In this case, and in view of the Treg tendency to accumulate after 3 cycles as observed in IMODALS (see Example 1 and Figure 2), lower cumulative daily doses of human IL-2 may be contemplated, such as a daily dose of 0.1 x10⁶ to 2x10⁶ IU, preferably 0.1 x10⁶ to 1.5x10⁶ IU, 0.1 x10⁶ to 1x10⁶ IU, or even 0.1 x10⁶ to 0.5x10⁶ IU. This type of treatment may notably be contemplated in ALS patient showing adverse effects of higher daily doses of human IL-2.

More generally, while each single dose should be comprised between 0.1 x10⁶ and 3x10⁶ IU, the clinician will know how to adapt the administration scheme in order to observe efficiency without unacceptable toxicity. In particular, starting from a given administration scheme (such as one of the schemes selected in IMODALS and MIROCALS clinical trials), a clinician will be able to monitor the number or frequency of Tregs, their immunosuppressive function, and/or the serum, plasma or cerebrospinal fluid (CSF) concentration of CCL2 and/or CCL17 and/or CCL18, as well as possible adverse events, and adapt the administration scheme in order to optimize the benefit to risk ratio (improving efficiency based on the number or frequency of Tregs, their immunosuppressive function, and/or the serum, plasma or cerebrospinal fluid (CSF) concentration of CCL2 markers and/or CCL17 and/or CCL18, and/or limiting drug-related adverse events).

In this context, in the context of the present disclosure, the treatment may comprise:
a) measuring Tregs number or frequency (in blood), and/or Tregs immunosuppressive function, and/or serum or plasma or CSF concentrations of CCL2, and/or serum, plasma or CSF concentrations of CCL17 and/or CCL18, at baseline from a biological sample of the subject (i.e on the day of starting the low dose human IL-2 treatment),
b) administering human IL-2 to the subject according to a first administration scheme according to the present disclosure comprising either repeated separated cycles of human IL-2 administration or continuous -metronomic- human IL-2 administration,
c) monitoring drug-related adverse events and measuring the same parameter(s) as at baseline from a biological sample of the subject taken 1-3 days following the end of a cycle of treatment or at least 7 days after continuous - metronomic - human IL-2 administration, and
d) continuing the first administration scheme when results are acceptable, or designing a second administration scheme depending on the results of step c).

In step d), the cycles or continuous daily dose may for instance be decreased or split into several lower single doses (instead of a once daily dose) if the ALS subject experiences poorly tolerated adverse events with compliance issues. If both the first and second administration schemes are based on cycles and the daily dose of human IL-2 during cycles is decreased, then the duration of each cycle may be increased to compensate. Alternatively, since lower daily doses are expected to be sufficient when using continuous administration rather than cycles, the second administration scheme may be based on continuous administration instead of cycles.

Conversely, if the ALS subject does not experience unacceptable toxicity but the therapeutic effect - as measured using Tregs number, frequency or immunosuppressive function (markers negatively correlated to disease progression) or CCL2, CCL17 and/or CCL18 plasma or CSF concentration (CCL2 is positively correlated to disease progression, CCL17 and/or CCL18 are indicative of a change of macrophage polarization from an pro-inflammatory M1 phenotype to an anti-inflammatory M2 phenotype) - is not sufficient, then the daily dose of cycles or continuous administration doses may be increased in order to increase chances that the ALS subject responds to the treatment, provided that each single dose administered to the subject is at most 3 x10⁶ IU. Since Tregs number, frequency or immunosuppressive function, and CCL2 plasma or CSF concentration have more particularly been correlated to disease progression, at least one of these markers will preferably be used. Because plasma or serum CCL2 is easier to measure and more reliable, CCL2 plasma or serum concentration is even more preferred.

CCL2 concentrations can be measured in plasma, or in serum or in CSF. The measurements can be performed on fresh or frozen (-20°C) plasma or serum or CSF samples. CCL2 concentrations are measured in fresh or frozen plasma or serum or CSF using solid phase immune-assay such as enzyme-linked immunosorbent assay (ELISA) method (as done in MIROCALS study) or cytometric beads assay (as done in IMODALS). IL2 unit dose increase would be considered when CCL2 concentrations, are over 80% of pretreatment baseline concentrations (ie, showing less than 20% decrease on treatment).

Whatever the selected administration scheme (which may be modified during treatment, as explained above), the treatment is preferably administered for the life-time of the subject or until unacceptable drug-related Serious Adverse Event.

### Other treatments not administered to the human subject

In the therapeutic uses and methods described herein the treatment does not comprise the administration of regulatory T cells to the subject. Indeed, contrary to what had been suggested by Thonhoff et al (Thonhoff JR et al. Neurol Neuroimmunol Neuroinflammation 2018; 5: e465), the inventors surprisingly found that the mere injection of low dose human interleukin 2 (ldIL-2) is sufficient to induce a significant improvement not only in Treg numbers but also most importantly in their suppressive function in all ALS subjects, without the need for Treg isolation and ex vivo expansion prior to re-injection. The treatment as described herein, which does not require ex vivo Tregs selection, expansion and re-injection, is thus much simpler and thus much less expensive and thus available to many more ALS subjects.

*"Regulatory T cells"* or *"Tregs"* are T lymphocytes having immunosuppressive activity. Natural Tregs are characterized by a CD4⁺CD25⁺Foxp3⁺ phenotype. Tregs are also characterized by their functional ability to inhibit proliferation of T-effector cells.

While human IL-2 may be administered to the ALS subject in combination with another treatment (see below), in addition to the lack of combined treatment with Tregs, the treatment as described herein is also not combined with the following treatments:
- In addition to the lack of combined treatment with Tregs, the human IL-2 administered to the subject is not complexed with anti-human IL-2 antibodies. Complexes of IL-2 with anti-IL-2 antibodies, rather than purified IL-2, have been used by Sheean et al in transgenic SOD1 ALS mouse, because the IL-2/IL-2 monoclonal antibody complexes were considered to increase the biological activity of IL-2, and because the selected anti-IL-2 monoclonal antibody clone permitted to confer specificity to cells expressing high-affinity αBγ IL-2R (CD25hiCD4+Foxp3+ Tregs and activated effector T-cells) rather than those expressing low-affinity Bγ IL-2R (memory CD8+ cells or natural killer cells) (Sheean RK et al. JAMA Neurol. 2018;75(6):681-689, see Methods, section "Data Collection From Animal Participants", paragraph 3).

However, the inventors have now shown that administration of low doses of non-complexed human IL-2 in ALS subjects is sufficient to specifically increase the number, proportion and suppressive function of Tregs (see Example 1).

As a result, in the context of the invention, human IL-2 administered to the subject is not complexed with anti-human IL-2 antibodies.
- In addition to the lack of combined treatment with Tregs, the treatment as described herein is also not combined with rapamycin or any other agent suppressive of effector T-cells (Teffs).

In the transgenic SOD1 ALS mouse, Sheean et al not only used IL-2 complexed with anti-IL-2 antibodies, but also combined complexed IL-2 with rapamycin treatment. Rapamycin is an immunosuppressant drug known to particularly suppress effector T-cells (Teffs) expansion, and has been used by Sheean et al. in combination with IL-2/anti-IL-2 complexes in order to specifically expand Treg cells with an activated phenotype and to exert immunosuppressive function, the combination being considered as essential because it inhibits proliferation of T effector cells, enabling selective expansion of Tregs (Sheean RK et al. JAMA Neurol. 2018;75(6):681-689, see Methods, section "Data Collection From Animal Participants", paragraph 3).

However, the inventors have now shown that mere administration of low doses of non-complexed human IL-2 in ALS subjects, without the combined administration of an agent immunosuppressive of Teffs such as rapamycin, is sufficient to specifically increase the number, proportion and function of Tregs (see Example 1).

As a result, in the context of the invention, in addition to the lack of combined treatment with Tregs, the treatment as described herein is also not combined with rapamycin or any other agent suppressive of effector T-cells (Teffs).

"Effector T cells" or "Teffs" include all CD4 cells other than Tregs. In particular, Teffs do not constitutively express FOXP3.
- In other words, in addition to the lack of combined treatment with Tregs, the human IL-2 administered to the subject is not complexed with anti-human IL-2 antibodies and the treatment as described herein is also not combined with rapamycin or any other agent suppressive of Teffs (as disclosed above).

### Other treatments preferably administered to the human subject

Currently, the only treatment available for the treatment of ALS is riluzole (2-Amino-6-(trifluoromethoxy)benzothiazole, CAS number 1744-22-5, trademark name Rilutek^{®}), a compound of formula:

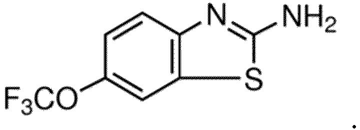

Approved for the treatment of ALS by the FDA in 1995, riluzole has been shown to be associated with a short median survival benefit of 2-3 months equating to a 9% absolute increase in 1-year survival (Miller RG, et al. Cochrane Database of Systematic Reviews 2012, Issue 3. Art. No.: CD001447).

While this short survival benefit is not satisfying, it is still better than no treatment and most ALS subjects are thus treated by riluzole.

Therefore, in a preferred aspect, the treatment as described herein with low dose human IL-2 preferably further comprises administering riluzole to said subject.

In ALS, riluzole is typically used at a daily dose of 100 mg/day by oral route, taken in two equal doses of 50 mg separated by about 12 hours. In case of toxicity, a lower daily dose, such as a daily dose of 50 mg/day by oral route, taken in two equal doses of 25 mg separated by about 12 hours, may be used.

When the treatment as described herein is combined with riluzole treatment, riluzole is thus preferably orally administered at a daily dose of 50 mg to 100 mg, taken in two equal doses of 25 mg to 50 mg separated by about 12 hours.

Further optional treatments commonly administered to ALS subjects may further be administered to the ALS subjects in the context of the invention, including antidepressants (when the ALS subject suffers from depressive symptoms), analgesics (to limit pain), anticholinergics (in case of hypersiallorhea), and antibiotics (in case of bacterial infection).

### Biological effects of the treatment

As presented in Example 1, the inventors have now shown that mere administration of low dose human IL-2 in human ALS subjects is able to:
- expand Tregs numbers/frequency and to improve their suppressive function (see in particular Figures 2 to 5 and Tables 4 and 5), and
- change the polarization of macrophages from a pro-inflammatory M1 phenotype to an anti-inflammatory M2 phenotype.

In particular, IMODALS clinical trial shows that mere administration of low dose human IL-2 in human ALS subjects is able to both:
∘ decrease the plasma concentration of the inflammatory chemokine CCL2 (see in particular **Figure 6A****).**
   CCL2 is a small inflammatory chemokine belonging to C-C subfamily also known as MCP1, which signals through the chemokine receptor-2 (CCR2), driving circulating leucocytes towards sites of neuroinflammation and which has been shown to correlate with disease score (Nagata T, et al. Neurol Res. 2007 Dec;29(8):772-6) and survival (Gille B et al. J Neurol Neurosurg Psychiatry. 2019 Dec;90(12):1338-1346).
   Decreasing CCL2 concentration is thus indicative of an effect on disease progression in ALS subjects.
∘ increase the plasma concentration of CCL17 and/or CCL18 (see in particular **Figure 6A** and **Figure 6B****).**
   CCL17 and CCL18 are chemokines mainly expressed by anti-inflammatory M2-polarized macrophages (Katakura T, et al. J Immunol. 2004 Feb 1;172(3):1407-13; Schraufstatter IU, et al. Immunology. 2012 Apr;135(4):287-98). Increasing CCL17 and/or CCL18 concentrations is thus indicative of a change in macrophage polarization from a pro-inflammatory M1 phenotype to an anti-inflammatory M2 phenotype.

Therefore, in the context of the present disclosure, the treatment described herein is preferably administered so that it:
- induces an increase in the number of Tregs and improves Tregs immunosuppressive function;
- induces a decrease of CCL2 plasma or serum or cerebrospinal fluid (CSF) concentration; and/or
- induces a shift in blood or central nervous system (CNS) of monocyte polarization towards M2 phenotype engaged in repair function, preferably evidenced by an increase of CCL17 and/or CCL18 plasma or serum or CSF concentration.

This may be achieved by:
- continuing the treatment as long as at least one of the above biological effects is obtained,
- adapting the administration scheme of human IL-2 (within the low doses scheme used in all the invention) in order to maintain at least one of the above biological effects (see section above relating to doses and administration schemes), and/or
- selecting for treatment ALS subjects in which administration of low dose human IL-2 (within the low doses scheme used in all the invention) results in at least one of the above biological effects.
In this case, the number and/or immunosuppressive function of Tregs, or the plasma or CSF concentration of CCL2, CCL17, or CCL18 or any combination thereof is(are) used as biomarker(s) for selecting ALS subjects benefiting from the low dose human IL-2 treatment, which are then treated with low dose human IL-2.

The following examples merely intend to illustrate the present invention.

### EXAMPLES

### Example 1: Immuno-modulation in Amyotrophic Lateral Sclerosis- a Phase II Study of Safety and Activity of Low Dose Interleukin-2 (IMODALS)

Low dose human IL-2 has been tested in ALS patients in the IMODALS phase 2 clinical trial (clinicalcaltrials.gov NCT02059759).

### Patients and Methods

### Study design and participants

This three-arm, randomized (1:1:1), double-blind, single-centre study of 2 doses of ld-IL-2 in parallel versus placebo included 36 ALS patients. The study protocol was approved by an independent ethics committee *(Le Comité de Protection des Personnes Sud Méditerranée III*; reference number: 2014.09.01 ter), declared on clinicalcaltrials.gov (NCT02059759) and was designed for adults less than 75 years old with probable, or laboratory-supported probable or definite ALS as defined by El Escorial Revised ALS diagnostic criteria (Brooks BR, et al. Amyotroph Lateral Scler Other Motor Neuron Disord 2000; 1: 293-9). The main inclusion criteria consisted of disease duration of less than 5 years, stabilised on riluzole treatment for over three months, and a vital capacity ≥ 70% of normal. Patients with severe cardiac or pulmonary disease, cancer, other life-threatening diseases, respiratory or feeding assistance, clinical signs of infection, positive serology (cytomegalovirus, Epstein Barr virus, or human immunodeficiency virus), auto-immune disorders (except asymptomatic Hashimoto thyroiditis), any clinically significant laboratory abnormality (excepting cholesterol, triglyceride and glucose), or other diseases precluding functional assessments were excluded, as were those who had received a vaccination in the 8 weeks preceding the first experimental dosing. All patients provided signed informed consent before entering the study.

### Randomisation and masking

Allocation was performed and blinding assured via a web-based inclusion and randomization (with blocking) application (a statistician otherwise not involved in the study prepared randomization lists). The size of blocks (3) remained undisclosed to all participants until unblinding. Clinical treatment unit (CTU) preparation and labelling were performed by a pharmacist who was the only unblinded trial participant. All laboratory assays were completely blinded. Samples were only identified by barcodes with the correspondence to randomisation number, time in the study (number of sampling time points=7), or treatment group (Placebo, 1MIU IL-2, 2MIU IL-2), unknown to the laboratory producing the data.

### Procedures

Upon inclusion by an investigating physician, baseline assessments were performed and included routine blood haematology and biochemistry, slow vital capacity, ALS Functional Rating Scale Revised (ALSFRS-R), chest x-ray, electrocardiogram (ECG) and thyroid function (Figure 1). Following randomisation (≤ 2 weeks before first administration on day 1), patients started a 5-day cycle of once-daily sub-cutaneous injections. 5-day cycles were repeated twice, on weeks 5 and 9, for a total of 3 cycles of treatment per patient (Figure 1). After the last treatment cycle, all patients were followed up for safety monitoring for a further 3 months.

Proleukin^{®} (aldesleukin) at 22 MIU vials was purchased from Novartis-pharma France. Clinical trial unit pharmaceutical preparation consisted of visually indistinguishable 1 ml polypropylene syringes containing 0·5 ml of either placebo (glucose for injection preparation D5% solution), or 1 MIU or 2 MIU of IL-2, according to randomisation. Assessments performed during the 6 months study period are indicated on Figure 1. Vital signs, concomitant medication and adverse events were assessed at each visit. Slow vital capacity was assessed according to current recommendations (https: / /www.encals.eu/outcome-measures).

### Clinical Immunophenotyping

Clinical flow cytometry was performed on fresh blood within 2 hr of phlebotomy. Peripheral blood was drawn into EDTA tubes and stained with two panels of monoclonal antibodies to identify CD3⁺, CD4⁺, CD8⁺ and regulatory T-Cells (Tregs; CD4⁺ CD25⁺ CD127^{low/-} FoxP3⁺), NK cells (CD16/56⁺), B lymphocytes (CD19⁺) and monocytes (CD14+). B cells, NK cells and CD3⁺ T cells are expressed as percentages of total lymphocytes; CD4⁺ and CD8⁺ T cells are expressed as percentages of CD3⁺ cells. Tregs and effector T-cells (Teffs) (calculated as the difference between CD4⁺ and Tregs), are expressed as percentages of CD4⁺ cells. Monocytes are expressed as a percentage of CD45+ leucocytes.

### Mechanistic Immunophenotyping

At each study visit, 20ml blood was collected into sodium heparin tubes and peripheral blood mononuclear cells (PBMC) isolated and cryopreserved. For analysis of Treg function, cryopreserved PBMC were thawed, stained with a cocktail of monoclonal antibodies (see below) and suppression assays established in V-bottom 96-well plates by co-culturing 500 sorted CD4⁺CD25^{-/lo}CD127⁺ effector T cells (Teffs) in the presence or absence of CD4⁺CD25^{high}CD127^{low} Tregs at various ratios (Treg:Teff 0:1, 1:2 and 1:1) with 1 × 10³ CD19⁺ B cells. Cells were stimulated with PHA (4 µg/ml; Alere) and incubated at 37°C, 5% CO₂, for 6 d. Proliferation was assessed by the addition of 0.5 µCi/well [³H] thymidine (PerkinElmer) for the final 20 h of coculture. Conditions were run in 6 replicates, and proliferation readings (counts per minute [CPM]) averaged. Any samples with averaged proliferation less than 3,000 CPM from the Teff wells alone were excluded. The percentage suppression in each culture was calculated using the following formula: percent suppression = 100 - [(CPM in the presence of Tregs ÷ CPM in the absence of Tregs) × 100]. All time points from an individual were analysed concurrently.

### Details of flow cytometry antibodies used in clinical immunophenotyping and mechanistic Immunophenotyping

The antibodies used in clinical immunophenotyping and Mechanistic Immunophenotyping as described in Table 1 below:

| **Clinical Immunophenotyping Tube 1** | | | |
|---|---|---|---|
| Marker | Fluorochrome | Clone | Supplier |
| CD3 | FITC | UCHT1 | Beckman Coulter |
| CD25 | PE | B1.49.9 | Beckman Coulter |
| CD4 | ECD | SFCI12T4D11 | Beckman Coulter |
| CD127 | PE-Cy7 | R34.34 | Beckman Coulter |
| FOXP3 | AF647 | 259D | Beckman Coulter |

| **Clinical Immunophenotyping Tube 2** | | | |
|---|---|---|---|
| Marker | Fluorochrome | Clone | Supplier |
| CD3 | FITC | UCHT1 | Beckman Coulter |
| CD56 | PE | N901 | Beckman Coulter |
| CD4 | ECD | SFCI12T4D11 | Beckman Coulter |
| CD8 | Pe-Cy5.5 | B9.11 | Beckman Coulter |
| CD16 | PE-Cy7 | 3G8 | Beckman Coulter |
| CD14 | APC | RMO52 | Beckman Coulter |
| CD19 | APC-AF750 | J3-119 | Beckman Coulter |
| CD45 | Krome Orange | J33 | Beckman Coulter |

| **Antibodies for Tregs suppression assay** | | | |
|---|---|---|---|
| Marker | Fluorochrome | Clone | Supplier |
| CD4 | APC-Cy7 | RPA-T4 | |
| CD14 | PerCP-Cy5.5 | HCD14 | |
| CD19 | Pacific blue | HIB19 | |
| CD25 | PE | 2A3 | |
| CD25 | PE | M-A251 | |
| CD127 | Alexa Fluor 647 | HIL-7R-M21 | |

### Transcriptomic analysis of Tregs activation markers in white blood cells

RNA was isolated from patient's blood using the LeukoLOCK Total RNA Isolation System (ThermoFisher). Whole blood was filtered through LeukoLOCK filters to capture the white blood cells, which were stored frozen (-80°C) in RNAlater. RNA was then extracted from these cells and quality and quantity measured. Subsequently, the RNA was converted to single stranded DNA and applied to Clariom D microarrays (Affymetrix) according to standard protocols. All the arrays were normalised and changes in transcript levels (gene expression levels) were determined using Transcriptome Analysis Console (TAC) (Affymetrix). Transcripts in the treated samples were defined as significantly differentially expressed from placebo if they were increased or decreased with a fold-change of 1.2 or more and a p-value = <0.05. Of the differentially expressed genes, several were associated with regulation and development of Tregs.

### Plasma cytokine determination

Plasma cytokine analysis was performed on -80°C frozen plasma samples. CCL2 and CCL17 plasma cytokine levels were measured by Multiplex bead assay (Luminex Human HS Cytokine Panel - R&D Systems), CCL18 by ELISA (Quantikine ELISA kit (DCL180B, R&D Systems).

### Plasma NfL determination

Plasma concentrations of the neurofilament light chain (NFL) were estimated using enzyme-linked immunosorbent assays (ELISA). More precisely, the quantitative determination of NF-light in human serum was undertaken by Meso Scale Discovery (MSD). The MSD GOLD plates (L45SA-1-MSD) were coated with 30 µl of capture antibodies (27016-UmanDiagnostics, 1:880 dilution), in 0.05 M carbonate buffer, pH 9.5 (w/v, 2/10000), at 4°C overnight. The plates were rinsed three times with 0.1% Tween 20/1X TBS washing buffer and then blocked with 100 µl 3% Milk/1X TBS at room temperature (RT) for 1 hour. Standards and samples were mixed with Heteroblock before loading (1:34 SD300 and 1:17 SD 600 respectively). After washing 25ul all samples, standards and quality controls were loaded in duplicate. Plates were then incubated for 1 hour (RT) on a shaker. After washing (3×5 mins), 25 µl of detector antibody (27017-UmanDiagnostics, 1:1282 dilution diluted in 1% Milk Wash buffer was added into each well and incubated for 1 hour on a shaker (RT). Plates were then washed (3×5 mins) before 25 µl Streptavidin SULFO-TAG (R32AD-5, MSD) per well were incubated for 1 hour on a shaker (RT). After washing (3×5 mins), 150 µl of 2X Read buffer was added into each well. The plate was read on the MSD machine. Each plate contained calibrators (0-1000 pg/mL) and quality controls. The interassay coefficients of variance were mostly below 10% and the mean intraassay coefficients of variance were below 10%.

Plasma NFL was also analysed using the Simoa method. NfL concentration in plasma was measured using an in-house ELISA on the Single molecule array platform (Quanterix, Lexington, MA), as previously described in detail (Gisslén M, et al. EBioMedicine. 2015 Nov 22;3:135-140). Samples were run in singlicates with a 4-fold dilution, which was compensated for in the final result output. Two QC samples were run in duplicates in the beginning and end of each run. For a QC sample with a concentration 10.9 pg/mL, repeatability was 3.7% and intermediate precision was 5.4%. For a QC sample with concentration 168 pg/mL, repeatability was 2.9% and intermediate precision was 3.4%. The measurements were performed by board-certified laboratory technicians who were blinded to clinical data.

### Outcomes

The primary pharmacodynamic outcome was the change in Tregs as a percentage of CD4+ T-lymphocytes on day 8 measured by clinical flow cytometry. Secondary pharmacodynamics were Treg number and percentage at all timepoints, including expression as incremental areas-under-the-curves (iAUC), and plasma levels of CCL2 and neurofilament light chain (NFL) as markers of disease activity. Exploratory analyses included measurements of number and frequency of leucocyte populations by flow cytometry as well as Treg cell functionality tests. Monocyte polarisation in response to treatment was investigated through analysis of their chemokine production profile (CCL17 and CCL18). Safety was assessed through a systematic check for predefined events (injection site reactions, flu-like symptoms, fatigue, gastro-intestinal signs, allergic reaction), abnormal vital signs, ECG results, chest x-rays, laboratory tests, and records of all adverse events reported during the study. As a secondary clinical outcome, changes in clinical function (ALSFRS-R and slow vital capacity-SVC) with time were assessed throughout the study.

### Sample size

Previous data (Thonhoff JR et al. Neurol Neuroimmunol Neuroinflammation 2018; 5: e465) demonstrated that 6 patients per group achieved 88% power to detect a 60% increase in Tregs at p α=0.05 (Mann & Whitney test). Because impact on disease activity is also of primary interest, we retained 12 patients per group to achieve 80% power at p α(2-tailed)= 0.05 for detecting a 40% decrease in plasma NFL at 3 and 6 months (Mann & Whitney test) based on a previous ALS study suggesting that this number might be sufficient to observe an effect on plasma NFL (Gaiottino J, et al. PloS One 2013; 8: e75091).

Interim safety data were evaluated once after the first 12 patients were included, had completed a first cycle of treatment and were then evaluated on day 8 (primary efficacy). This report remained undisclosed until the end of the study.

### Statistical analyses

Categorical variables are described as absolute and relative frequencies. Quantitative variables are summarized by mean, median, standard deviation and range. Flow cytometry parameters were analysed as changes from baseline at D8 (primary criteria) and D64, i.e. absolute differences between each time point and baseline D1; Overall changes in immune cells over time measures of first cycle (D1, D8, D29) and third cycle (D57, **D64,** D85) were summarised as incremental time-normalised areas-under-the-curves (iAUC, using the trapezoidal method), minus D1 or D57 value respectively; iAUCt for trough values were calculated using values measured at D1, D29, D57 and D85 minus D1. Eosinophils count were analysed in the same way as cytometry parameters. ALSFRS-R measures were summarised by regression slopes from D1 to D85. For SVC and NFL, absolute differences between D85 and baseline D1 were analysed. For CCL2, CCL17 and CCL18 baseline normalised values at D64 were analysed.

Kruskall-Wallis tests were used to test for differences between the three study arms. If a significant difference at p<0.05 was detected, Mann-Whitney tests were used for pairwise comparisons to pinpoint the detected differences.

Dose-response relationship on summary measures were analyzed using one-way Anova assessing whether the change in outcome variable was constantly increasing across levels of doses (linear trend test).

### Results

### Conduct of study

Between September 21st and December 4th, 2015, thirty-nine patients were screened. Of the latter, 3 were excluded and 36 randomized **(****Figure 1****).** After 12 inclusions and 1 cycle of treatment, the independent data safety monitoring board found no safety concerns, and inclusions continued. With only one exception (see **Figure 1****),** all randomized patients fully completed the 3 cycles of treatment over 3 months and 3-month post treatment follow-up. All 36 randomized patients were included in the intention-to-treat and safety populations **(****Figure 1****).** Of 252 maximum possible assessments for clinical and laboratory measurements for primary/secondary outcomes in the trial, all but one were available for analysis. **(****Figure 1****).**

### Patients characteristics and disease history

Though baseline differences were not statistically significant, patients in the 2MIU group had a higher female-to-male ratio, and slightly more severe disease features (see **Table 2** below with respect to clinical parameters, **Table 4** (column D1) for immune cell parameters expressed as absolute numbers at baseline and **Table 5** - for immune cell parameters expressed as frequencies, and **Table 6** for neurofilaments). Nonetheless, no clear imbalance that would influence the results was identified between groups.

**Table 2. Demographic and clinical baseline characteristics of study participants**

| | | Placebo (n=12) | IL2 at 1 MIU/d (n=12) | IL2 at 2 MIU/d (n=12) |
|---|---|---|---|---|
| Age | | | | |
| | Mean (SD) | 56·45 (9·57) | 54·98 (10·99) | 57·68 (12·91) |
| | Median (Range) | 56·20 (42·2 to 69·7) | 54·80 (40·2 to 75·4) | 61·25 (36·5 to 76·6) |
| Sex (female) | | 3 (25%) | 5 (41·7%) | 3 (25%) |
| BMI | | | | |
| | Mean (SD) | 26·80 (5·6) | 25·34 (2·53) | 24·39 (1·71) |
| | Median (Range) | 25·10 (22·2 to 43·4) | 24·90 (21·90 to 28·7) | 24·35 (21·6 to 26·7) |
| Age at onset | | | | |
| | Mean (SD) | 54·27 (9·85) | 52·43 (11·02) | 55·80 (12·86) |
| | Median (Range) | 55.30 (38.1 to 68.0) | 52.20 (37.4 to 72.6) | 58.25 (35.1 to 76.0) |
| Disease duration (years) | | | | |
| | Mean (SD) | 2·2 (1·44) | 2·60 (1·33) | 1·96 (1·44) |
| | Median (Range) | 1·75 (0·5 to 5·0) | 2·85 (0·9 to 4·6) | 1·45 (0·6 to 4·6) |
| Duration of riluzole treatment (months) | | | | |
| | Mean (SD) | 16·58 (12·49) | 20·70 (14·90) | 14·18 (11·47) |
| | Median (Range) | 12·35 (4·6 to 39·8) | 17·45 (5·0 to 45·1) | 11·10 (3·1 to 34·0) |
| Diagnosis | | | | |
| | Definite | 5 (41-7%) | 6 (50%) | 4 (33·3%) |
| | Probable | 5 (41-7%) | 6 (50%) | 3 (25%) |
| | Probable - laboratory supported | 2 (16·7%) | 0 (0%) | 5 (41·7%) |
| Familial form | | 0 | 2 (16-7%) | 2 (16-7%) |
| Site of onset | | | | |
| | Limb | 11 (92%) | 11 (92%) | 9 (75%) |
| | Bulbar | 1 (8%) | 1 (8%) | 3 (25%) |
| Slow vital capacity (percentage predicted) | | | | |
| | Mean (SD) | 94·4 (12·4) | 101·5 (18·1) | 93·6 (16·3) |
| | Median (Range) | 96·5 (77·00 to 119·00) | 101·0 (79·00 to 132·00) | 94.5 (72·00 to 118·00) |
| ALSFRS-R score | | | | |
| | Mean (SD) | 38·8 (3·4) | 38·0 (4·8) | 37·8 (5·3) |
| | Median (Range) | 38·5 (34·00 to 45·00) | 38·0 (30·00 to 44·00) | 39·5 (26·00 to 44·00) |

| | | | | |
|---|---|---|---|---|
| Categorical data are presented as number (%). ALSFRS = Amyotrophic Lateral Sclerosis Functional Rating Score - Revised. BMI = Body Mass Index. SD = standard deviation. | | | | |

### Safety and Tolerability

Clinical tolerance was satisfactory at both doses of IL-2. During the entire follow-up (D1-D169), no drug-related serious adverse event (SAE) occurred and most drug-related non serious adverse events (NSAEs) were transient and of mild to moderate grades (see **Table 3** below).

**Table 3. Safety- Number of patients (frequency) presenting adverse events during treatment cycles.**

| | **TREATMENT GROUP** | | | **Total (N=36)** |
|---|---|---|---|---|
| **Adverse Events** | **2MIU (N=12)** | **1MIU (N=12)** | **Placebo (N=12)** | |
| Injection site reactions | 12 (100%) | 11 (91.7%) | 1 (8.3%) | 24 (66.7%) |
| Flu-like symptoms | 3 (25.0%) | 0 | 0 | 3 (8.3%) |
| Fatigue | 2 (16.7%) | 1 (8.3%) | 2 (16.7%)0 | 5 (13.9%) |
| Gastro-intestinal signs | 2 (16.7%) | 1 (8.3%) | 0 | 3 (8.3%) |
| Rhinitis | 2 (16.7%) | 0 | 0 | 2 (5.6%) |
| Nasopharyngitis | 1 (8.3%) | 1 (8.3%) | 0 | 2 (5.6%) |
| Headache /Migraine | 4 (33.3%) | 0 | 0 | 4 (11.1%) |
| Chest pain | 1 (8.3%) | 0 | 0 | 1 (2.8%) |
| Cold sweat | 0 | 1 (8.3%) | 0 | 1 (2.8%) |
| Arthralgia | 1 (8.3%)0 | 0 | 0 | 1 (2.8%) |
| Myalgia | 0 | 0 | 1 (8.3%) | 1 (2.8%) |
| **Total** | **12 (100%)** | **11 (91.7%)** | **3 (25.0%)** | **26 (72.2%)** |

During the treatment period (D1-D85), frequencies of patients presenting NSAEs during cycles were higher in the IL-2 groups, n= 11 (91·7%) and n=12 (100%) at the 1 and 2 MIU/day doses respectively, compared to n= 3 (25.0%) with placebo (Table 3). Local reactions at injection sites (erythema, pain) were the most common NSAEs of comparable frequency in the 2 active treatment groups (all patients except one presented injection site reactions) while only one patient reported such an event in the placebo group. Flu-like symptoms (including myalgia, chills, fever, arthralgia), which are characteristic of Il-2 treatment (Hartemann A et al. Lancet Diabetes Endocrinol 2013; 1: 295-305), were reported only at the 2 MIU/day dose (25%). One patient in the 2 MIU/day group withdrew from treatment after 2 days of treatment in the 3^{rd} cycle because of severe flu-like symptoms not responding to corrective therapy (see **Figure 1****).**

Outside treatment cycles, only one case of nausea/vomiting was imputed to treatment among other AEs. One patient (1MIU/day group) with a history of prostatic adenoma developed severe urinary retention 10 days after the last administration and required hospitalisation for prostatic surgery. Other events were related to ALS disease or other pre-existing conditions.

No abnormalities were observed among the routine laboratory parameters, except for an elevation of CRP at day 8 in the one patient presenting flu-like symptoms in the 2 MIU/day group, and another at D57 in relation to a viral infection. As for haematology parameters, there was no significant change observed except for eosinophil counts that were significantly increased compared to placebo at day D8 and D64 in the 2MIU/day group (see **Tables 4 and 5** below); changes of a lesser degree were observed at 1MIU/day and were significant only at D64. In the 2MIU/day group, 3 patients presented eosinophil increases above 1·5x10⁹/l, but remained asymptomatic; all counts were close to baseline values at D169 (no significant differences between groups) and all within normal range.

**Table 4. Immune cell parameters at baseline (D1) and response. Results expressed as absolute count / mm³: mean (SD). * p< 0.05, ^{**} p<0.01, ^{***} p<0.001, ^{****} p<0.0001**

| | **Dose group** | **Baseline D1** | **D8 change from D1** | **D64 change from D1** | **iAUC trough D29-D57-D85** | **D169 change from D1** |
|---|---|---|---|---|---|---|
| **CD8** | **2MIU** | 308.1 (147.2) | 94.8 (140.2)^{*} | 111.4 (124.6)^{**} | 40.4 (115.6) | (78.4) |
| | **1MIU** | 316.0 (146.1) | 36.3 (43.6) | 25.3 (28.9)^{*} | 0.3 (22.0) | 18.2 (73.9) |
| | **Placebo** | (138.5) | -18.3 (59.5) | -8.3 (38.6) | -26.7 (35.2) | -26.0 (45.0) |
| **Treg** | **2MIU** | 66.0 (29.7) | 123.4 (76.1)^{****} | 175.9 (130.3)^{**} | 19.7 (26.4)^{***} | 8.9 (23.8) |
| | **1MIU** | 66.7 (12.6) | 62.9 (29.1)^{****} | 68.3 (23.6)^{****} | 5.0 (9.1)^{**} | 6.8 (14.9) |
| | **Placebo** | 52.2 (17.6) | -2.4 (9.7) | -8.5 (14.2) | -5.9 (5.6) | -2.3 (11.1) |
| **Teff** | **2MIU** | 801.2 (308.2) | 331.9 (265.3)^{***} | 483.3 (316.6)^{** **} | 141.3 (238.3)^{*} | 119.9 (191.3) |
| | **1MIU** | 853.2 (232.7) | 180.8 (140.1)^{**} | 189.6 (173.9)^{**} | 39.5 (81.2)^{*} | 63.7 (144.4) |
| | **Placebo** | 704.5 (241.3) | -8.8 (90.8) | 13.9 (84.6) | -38.6 (74.8) | -7.5 (63.6) |
| **Ratio (%) Treg/ Teff** | **2MIU** | (2.90) | 7.98 (3.32)^{****} | (6.03)^{****} | 0.51 (1.45)^{***} | (1.26) |
| | **1MIU** | (2.10) | 4.87 (1.71)^{****} | 5.44 (2.02)^{****} | 0.12 (0.99) | 0.12 (1.05) |
| | **Placebo** | 7.63 (1.76) | -0.56 (1.42) | -1.32 (1.40) | -0.54 (0.35) | -0.29 (1.66) |
| **NK** | **2MIU** | 171.0 (70.5) | 109.3 (49.6)^{***} | 115.4 (62.4)^{****} | 29.5 (39.2)^{*} | (35.0) |
| | **1MIU** | 222.2 (129.5) | 78.5 (57.5)^{***} | 86.1 (64.8)^{***} | 18.1 (41.0) | (73.5) |
| | **Placebo** | 226.1 (202.2) | -23.8 (46.9) | -12.5 (41.8) | -24.0 (59.7) | (140.2) |
| **CD19** | **2MIU** | 264.9 (230.6) | -23.6 (83.3) | 1.33 (57.1) | -0.1 (93.0) | 25.3 (72.8) |
| | **1MIU** | 232.8 (62.9) | -16.5 (49.8) | -25.1 (39.1) | -8.4 (22.5) | 14.3 (30.4) |
| | **Placebo** | 205.6 (113.5) | -14.4 (50.8) | -16.8 (41.2) | -20.4 (35.0) | -13.5 (49.6) |
| **Monocytes** | **2MIU** | (147.1) | -23.2 (89.0) | -50.7 (119.7) | -41.5 (74.7) | -52.1 (96.2) |
| | **1MIU** | 391.9 (120.8) | 36.2 (57.9) | -25.1 (52.8) | -2.7 (54.0) | -7.0 (61.9) |
| | **Placebo** | 454.4 (227.4) | -5.7 (88.8) | 10.4 (91.4) | -1.8 (63.6) | -23.9 (141.3) |
| **Eosinophils** | **2MIU** | 172.5 (78.6) | 236.7 (92.8)^{***} | 590.0 (398.0) ^{***} | 134.7 (83.5)^{***} | 45.5 (93.6) |
| | **1MIU** | 152.5 (89.7) | 67.5 (52.6) | 155.8 (148.0)^{**} | 35.0 (33.9)^{*} | -18.3 (57.2) |
| | **Placebo** | (76.9) | 31.7 (43.7) | 23.3 (76.1) | 8.2 (32.5) | 33.3 (53.3) |

**Table 5. Immune cell parameters at baseline (D1) and response. Results expressed as frequencies: mean (SD). ^{a} total leucocytes from haematology lab. ^{*} p< 0.05, ^{**} p<0.01, ^{***} p<0.001, ^{****} p<0.0001**

| | **Dose group** | **Baseline D1** | **D8 change from D1** | **D64 change from D1** | **iAUC trough D29-D57-D85** | **D169 change from D1** |
|---|---|---|---|---|---|---|
| **CD8 (% CD3+)** | **2MIU** | 25.0 (8.9) | -3.1 (1.7)^{**} | -4.3 (2.6)^{**} | -1.4 (1.1) | -1.4 (1.4) |
| | **1MIU** | 24.1 (6.2)) | -2.0 (1.2)* | -2.7 (1.3)^{**} | -1.0 (0.6) | -0.6 (1.4) |
| | **Placebo** | 26.1 (9.0) | -0.4 (2.3) | -0.3 (2.5) | -0.9 (1.5) | -1.5 (2.4) |
| **Treg (% CD4+)** | **2MIU** | 7.6 (2.4) | 6.2 (2.2)^{****} | 7.6 (3.9)**** | 0.4 (1.2)^{***} | 0.2 (1.0) |
| | **1MIU** | 7.6 (1.8) | 3.9 (1.2)^{****} | 4.4 (1.5)**** | 0.1 (0.9) | 0.1 (0.9) |
| | **Placebo** | 7.1 (1.5) | -0.5 (1.3) | -1.2 (1.2) | -0.5 (0.3) | -0.3 (1.4) |
| **NK (% CD45)** | **2MIU** | 2.4 (1.0) | 1.4 (0.9)^{***} | 1.2 (0.9)^{***} | 0.4 (0.5) | 0.2 (0.5) |
| | **1MIU** | 3.2 (2.1) | 1.1 (1.0)^{**} | 1.4 (0.8)^{***} | 0.2 (0.5) | 0.1 (0.9) |
| | **Placebo** | 3.6 (2.4) | -0.2 (0.8) | -0.1 (0.7) | -0.2 (0.6) | -0.6 (1.4) |
| **CD19 (% CD45)** | **2MIU** | 3.5 (2.0) | -0.3 (0.9) | -0.2 (1.1) | -0.1 (0.8) | 0.3 (1.2) |
| | **1MIU** | 3.3 (1.0) | -0.3 (0.4) | -0.2 (0.2) | -0.1 (0.3) | 0.3 (0.5) |
| | **Placebo** | 3.4 (1.8) | -0.1 (0.9) | -0.2 (0.6) | -0.2 (0.5) | -0.1 (0.4) |
| **Monocytes (% CD45)** | **2MIU** | 6.7 (1.2) | -0.6 (1.4) | -1.4 (1.3)^{**} | -0.7 (1.3) | -0.9 (1.7) |
| | **1MIU** | 5.4 (1.2)* | 0.5 (0.5) | -0.04 (0.6) | 0.1 (0.5) | -0.1 (0.5) |
| | **Placebo** | 7.3 (2.5) | 0.2 (1.0) | 0.1 (1.0) | 0.2 (0.6) | -0.2 (0.8) |
| **Eosinophils (% CD45)^{a}** | **2MIU** | 2.7 (1.8) | 3.3 (1.5)^{***} | 6.8 (4.1) ^{***} | 1.8 (1.0)^{***} | 0.4 (1.3) |
| | **1MIU** | 2.3 (1.8) | 1.0 (0.8) | 2.5 (2.2)^{**} | 0.6 (0.5) | -0.2 (0.7) |
| | **Placebo** | 2.3 (0.9) | 0.6 (0.7) | 0.4 (1.2) | 0.2 (0.6) | 0.6 (1.0) |

### Ld IL-2 effects on peripheral blood mononuclear cells

The *a priori* defined primary pharmacodynamic outcome of an increase in the frequency of Tregs as a percentage of CD4+ T-lymphocytes at day 8 was highly significant (p<0.0001 by the Mann-Whitney U test) for both the 2 MIU and 1 MIU arms (2MIU: mean [SD]: +6.2% [2.2]; 1MIU: mean [SD]: +3.9% [1.2]) as compared to placebo (mean [SD]: -0.5% [1.2]) see **Table 4** above and **Figures 2A-B****).** The effect size was large for both IL-2 groups: 2MIU ES=3.7 (IC95%: 2.3-4.9); 1MIU ES=3.5 (IC95%: 2.1-4.6). Furthermore, when examining the change in Treg frequency from baseline, a clear distinction was observed in all IL-2 recipients (increase range 23 to139%) with no overlap with placebo group (change range -51 to 9%) **(****Figure 2B****).** Secondary outcome for Tregs revealed that the frequency and absolute count significantly increased compared to baseline and placebo during subsequent treatment cycles **(****Figures 2A-D****, Tables 4 and 5** above). Furthermore, the peak during cycle 3 was higher than that observed during cycle 1, suggesting that successive treatment cycles have residual effects that might be cumulative. This suggestion is further supported by significantly higher iAUC trough T_{reg} levels (measuring the residual Treg change before beginning a new cycle) in IL2 arms as compared to placebo **(****Figures 2E-F****, Tables 4 and 5** above). In general, the 2MIU arm resulted in higher T_{reg} peaks and trough levels than the 1MIU arm. Ld IL2 also resulted in a moderate increase in the frequency and number of NK cells for both IL-2 groups (maximum 1.7 fold increase in number at D64 for the 2MIU group); an increase in the number of CD8 T cells for both IL-2 groups (maximum 1.4 fold increase in number at D64 for the 2MIU group); an increase in the number of CD4 Teff for both IL-2 groups (maximum 1.6 fold increase in number at D64 for the 2MIU group) and a decrease in the frequency of monocytes in the 2MIU group at d64 (all data shown in **Tables 4 and 5** above).

Exploratory analysis of Treg phenotype and function was performed using cryopreserved PBMC focusing primarily on responses at baseline and following 3 cycles of treatment (day 1 and day 64). We observed good correlation between the frequency of Tregs defined in blood by clinical cytometry and when sorting Tregs from cryopreserved PBMC (R²=0·91, p<0·0001). Similar to results in fresh blood, analysis of cryopreserved PBMC revealed a significant increase in the frequency of Tregs following 3 cycles of IL-2 treatment **(****Figure 3A-C****).** Furthermore, batched analysis of all time points from a single individual on the same day allowed direct comparison of CD25 expression before and after treatment and revealed a dose dependent increase in expression on Tregs in response to treatment (CD25 median MFI and range at D1 vs D64: 2 MIU= 4651, range 2892-5886 vs 9015, range 4220-14446, p=0·002; 1 MIU= 4230, range 3388-5423 vs 7778, range 5564-9037, p=0·001; placebo = 4105, range 2992-5656 vs 3867 range 1923-5669, p=0·83 ; **Figure 4A-C****).** A smaller, but still significant increase in CD25 expression, was also observed on effector T cells (CD25 median MFI and range at D1 vs D64: 2 MIU= 426 range 116-897 vs 528 range 122-948, p=0·02; 1 MIU= 336 range 132-478 vs 362 range 124-577, p=0·001; placebo= 251 range 195-919 vs 263 range 158-881, p=0·24, **Figure 3D-F****).** Treg function was assessed by *in vitro* co-culture assays using effector T cells from the corresponding time point as responder cells. In cultures lacking Tregs, we observed no effect of IL-2 administration on the proliferation of responder T cells **(****Figure 3G-I**). However, we did observe an increase in suppressive function of Tregs following 3 cycles of IL-2 therapy, which reached statistical significance for the 1 MIU dose (Median % suppression, range at D1 vs D64: 2 MIU = 53%, range 18-88% vs 76%, range 19-91%, p=0·06; 1 MIU = 65%, range 23-84 vs 80%, range 36-97% p=0·001; **Figure 4D-E****).** In contrast, we observed a slight decrease in Treg function in the placebo group (Median % suppression, range at d0 vs d64: placebo= 73% range 53-95% vs 59% range 32-97%; p=0·07; **Figure 4F****).** When comparing the percent change in Treg suppressive function over the treatment period (relative to suppression at baseline), we observed a significant difference between both groups treated with IL-2 when compared to placebo (2 MIU vs placebo, p=0·008; 1 MIU vs placebo, p=0·005; **Figure 4G****).** We also assessed the relationship between the change in Treg frequency and CD25 expression with the change in Treg suppressive function in response to treatment for each individual. We observed a highly significant correlation between these measurements with individuals clustering based on treatment group (Treg frequency, p=0·0001, R²=0·42; Treg CD25 mfi, p=0·001, R²=0·33, **Figure 4H-I**).

### Ld IL-2 effects on white blood cells transcriptome

The transcriptomic data obtained in white blood cells at D64 are presented in **Figure 5** and show that there is an increase in the gene expression of the following Treg activation markers (FOXP3, CTLA4, IKZF2 and IL2RA) in the white blood cells. There is a dose-dependent increase in expression of each of these genes when patients are treated with 1MIU or 2MIU compared to the untreated (placebo) group following 3 cycles of low-dose IL-2 treatment at Day 65.

### Ld IL-2 effects on plasma cytokine concentrations

We assessed plasma levels of cytokines/chemokines previously reported to be elevated in individuals with ALS (CCL2) or those associated with macrophage/microglial polarization (CCL17 and CCL18). Following the third treatment cycle (D64), we observed a difference between the three groups in the plasma levels of CCL2 (p=0.005, **Figure 6A****),** both active dose groups showing a dose dependent changes of CCL2 level, which was significantly reduced compared to placebo with the 2 MIU dose (p=0.005) though not reaching statistical significance with the 1 MIU dose (p=0.06). We also observed a difference between treatment groups at D64 in CCL17 and CCL18 (p=0.00001 and 0.0028 respectively, **Figure 6B-C****)** with an increase being observed in both treatment groups compared to placebo (CCL17: 2MIU p=0.0001 and 1MIU p=0.0138; CCL18: 2MIU p=0.0012 and 1MIU p=0.0094).These results suggest that ld IL-2 treatment was associated with a decrease of the inflammatory marker CCL2 associated with ALS and a concomitant shift of monocytes towards the M2 phenotype.

### Ld IL-2 effects on disease progression as assessed through changes over time (D1-D85) with ALSFRS-R, Slow Vital Capacity and plasma-NFL

There were no significant differences among the three groups with regards to time related changes in the ALSFRS-R score (Kruskal Wallis =4·25, p= NS), Slow Vital Capacity (Kruskal Wallis= 1·07, p= NS), or plasma NFL levels (Kruskal Wallis= 0·34, p= NS). For NFL, reanalysis of the plasma samples using the SIMOA approach provided similar results (Kruskal Wallis= 2·44, p= NS). However, in the overall population, none of these parameters showed statistically significant changes over the 3 month treatment period - ALSFRS-R (points/month) mean slope [95% CI]= -0.8 [-2·4, +0·8]; SVC (percent predicted) mean change at d85 from d1 [95% CI]= -2·2 [-23·4, +19·0]; NFL-Elisa D85 change from D1, (pg/ml) mean change [95% CI] = + 0·63 [-62·5, + 63·7]; NFL-SIMOA D85 change from D1, (pg/ml) mean change [95% CI] = -1·64 [-26·25, +22·97]- demonstrating that these parameters were poorly sensitive to change over a three-month time period.

Moreover, with respect to plasma NFL levels, the high variability of baseline levels in all treatment arms (see **Table 6** below) probably prevented any significant observation for the selected sample size.

**Table 6. Baseline neurofilament light chain blood levels. SD = standard deviation.**

| | Placebo (n=12) | IL2 at 1 MIU/d (n=12) | IL2 at 2 MIU/d (n=12) |
|---|---|---|---|
| NFL: neurofilament light chain (units) | | | |
| Mean (SD) | 127.84 (89.90) | 135.55 (76.80) | 178.19 (94.84) |
| Median (Range) | 116.6 (6.7 to 349.2) | 103.4 (46.2 to 245.5) | 144.1811 (109.1 to 460.0) |

Despite the lack of significant observation, there is a trend in changes in plasma levels at D85 suggesting that, while plasma NFL levels tend to increase in the placebo group (suggesting some progression of disease), it is not the case for ALS patients treated with the 1 or 2 MIU dose (see **Figure 7****).**

### Conclusions

First, our results show that ld IL-2 at two doses was clinically well tolerated in ALS subjects over three cycles, and no further safety issues were detected following treatment withdrawal. In keeping with previous reports (Koreth J et al. N Engl J Med 2011; 365: 2055-66 ; Saadoun D et al. N Engl J Med 2011; 365: 2067-77 ; Hartemann A et al. Lancet Diabetes Endocrinol 2013; 1: 295-305 ; Castela E et al. JAMA Dermatol 2014; 150: 748-51), our findings clearly show that ALS patients, who are particularly vulnerable to treatment toxicity, can withstand repeated cycles of treatment with ld IL-2. The safety of ld IL-2 is further supported by the lack of significant deterioration in ALSFRS-R or SVC over the treatment period across all groups, and within groups, including placebo. Second, we observed a significant, dose dependent increase in both the absolute number and relative frequency of Tregs in both the 2MIU and 1MIU groups. Comparing these results to those obtained in a double-blind randomised clinical trial in individuals with Type 1 Diabetes (T1D; Hartemann A et al. Lancet Diabetes Endocrinol 2013; 1: 295-305), we observed a similar magnitude of Treg response to ld IL-2, with around 1·5-fold increase in Treg percentage of CD4 cells (Treg frequency) following 5 days of treatment at 1MIU. Of note, all individuals in both groups on active treatment showed an increase in Treg number and frequency. Thus, although it has been suggested that Tregs from ALS patients may have impaired endogenous responsiveness to IL-2 (Thonhoff JR et al. Neurol Neuroimmunol Neuroinflammation 2018), potentially making them unresponsive to ld IL-2 treatment, in this cohort of ALS patients we observed no evidence of intrinsic impairment in Treg responsiveness to ld IL-2. This is very important, since this means, that contrary to what had been suggested by Thonhoff et al, it is possible to significantly expand ALS patients' Tregs by mere administration of ldIL-2, without the need for prior isolation of the patient's Tregs, ex vivo expansion and re-injection to the patient. Due to its simplicity and much lower cost, it will be possible to make the proposed treatment based on simple administration of ldIL-2 available to a much higher number of ALS patients.

Third, we have shown that the increase in Treg response was sustained over 4 weeks following a 5-day treatment cycle. This is important as optimum clinical efficacy is likely to require a sustained increase in Treg levels. We selected our treatment schedule based on the study in T1D (Hartemann A et al. Lancet Diabetes Endocrinol 2013; 1: 295-305), and the present study confirms a significant expansion in Treg number and frequency at trough level (i.e. before the start of the next treatment cycle) and also that this expansion increases with repeated cycles. However, it remains to be seen whether our treatment schedule is the most effective for controlling neuro-inflammation in ALS, or whether different treatment schedules (e.g., more frequent administration of ld IL-2) will be more useful therapeutically.

Taken together our findings suggest that in this ALS cohort there was no loss of sensitivity to ld IL-2 with repeated administration. Consistent with this and in agreement with other reports (Todd JA, et al. PLoS Med. 2016 Oct 11;13(10):e1002139; Hirakawa M, et alJCI Insight. 2016 Nov 3;1(18):e89278), we observed that ld IL-2 leads to a preferential increase in expression of CD25 on Tregs. This may increase sensitivity of Treg cells to both administered and endogenous IL-2, thus potentially enhancing and sustaining any treatment effect.

In order to understand the relevance of the change in Treg number to any treatment effect, we assessed Treg function before and after ld IL-2 administration. Indeed, in ALS patients, Tregs have been shown not only to be reduced in numbers, but also to express lower levels of FOXP3 (Henkel JS et al. EMBO Mol Med 2013; 5: 64-79)and to be dysfunctional (reduced immunosuppressive function, Beers DR et al. JCI Insight. 2017;2(5):e89530), and both the decreased level of FOXP3 and the reduced immunosuppressive function have been correlated to disease progression (Beers DR et al. JCI Insight. 2017;2(5):e89530; Thonhoff JR et al. Curr Opin Neurol 2018; 31: 635-9). We used an autologous co-culture assay to measure the ability of FACS-isolated Tregs to suppress the proliferation of CD4 effector T cells. Our results demonstrate that, overall, treatment with ld IL-2 results in both an increase in Treg frequency and in Treg suppressive function. This improvement in Treg function is highly significant in the 1MIU group, with all individuals showing an increase in function. In the 2MIU group, only a trend toward significance (p=0·06) was observed, due to increased variation in response between individuals (Figure 4). The dual efficacy of ld IL-2 is illustrated by the highly significant correlation between increased Treg frequency and function, though we also observed individual variation in treatment response with some treated individuals showing a more significant change in either Treg frequency or Treg function and others showing an increase in both. Conversely, individuals in the placebo group tended to lose either Treg frequency or function during the same timeframe.

In addition, the transcriptomic data obtained in white blood cells at D64 show that there is an IL-2 dose-dependent increase in the gene expression of the following Treg activation markers (FOXP3, CTLA4, IKZF2 and IL2RA) in the white blood cells. These results further strengthen our observation that ld IL2 administration in ALS patients enhances Tregs immunosuppressive function. Moreover, since FOXP3 expression level in Tregs has been shown to correlate with disease progression (Beers DR et al. JCI Insight. 2017;2(5):e89530; Thonhoff JR et al. Curr Opin Neurol 2018; 31: 635-9), these results further support therapeutic efficiency of low dose human IL-2 administration in ALS patients.

With regard to effects of ld IL-2 on blood markers of ALS disease activity, we found a significant and dose-dependent reduction in the plasma concentration of CCL2, a small chemokine belonging to C-C subfamily which signals through the chemokine receptor-2 (CCR2), driving circulating leucocytes towards sites of neuroinflammation. CCL2 knockout mice have reduced infiltration of circulating leucocytes at sites of neuroinflammation and resistance to disease in models of autoimmunity and inflammation, suggesting this pathway plays a role in driving pathogenesis. Moreover, elevated CCL2 expression levels have been observed in neural tissue from individuals with ALS and expression is associated with infiltration and activation of macrophages and microglia (Henkel JS, et al. Ann Neurol. 2004 Feb;55(2):221-35; Baron P, et al. Muscle Nerve. 2005 Oct;32(4):541-4). CCL2 levels in biological fluids are also elevated in individuals with ALS (Martinez HR, et al. Neurologia. 2017 Oct 10. pii: S0213-4853(17)30280-3; Gille B et al. J Neurol Neurosurg Psychiatry. 2019 Dec;90(12):1338-1346, Gupta PK, et al. J Neuroinflammation. 2011 May 13;8:47; Kuhle J, et al. Eur J Neurol. 2009 Jun;16(6):771-4; Baron P, et al. Muscle Nerve. 2005 Oct;32(4):541-4) and have been shown to correlate with disease score (Nagata T, et al. Neurol Res. 2007 Dec;29(8):772-6) and survival (Gille B et al. J Neurol Neurosurg Psychiatry. 2019 Dec;90(12):1338-1346), indicating that CCL2 is a useful biomarker of disease activity. Therefore, the observation of a significant and dose-dependent reduction in the plasma concentration of CCL2 in IMODALS further supports therapeutic efficiency of low dose human IL-2 administration in ALS patients.

Interestingly, ld IL-2 treatment was also associated with a significant increase of plasma levels of CCL17 and CCL18, in keeping with a change in macrophage/microglial polarisation towards an anti-inflammatory M2-like phenotype (Katakura T, et al. J Immunol. 2004 Feb 1;172(3):1407-13; Schraufstatter IU, et al. Immunology. 2012 Apr;135(4):287-98). Overall, the changes in inflammatory biomarkers of macrophage activation and polarisation are consistent with a role of ld IL-2 in controlling cytopathic microglial activation associated with ALS progression.

Tregs are known to influence macrophage activation and polarisation, (primarily towards an M2-like phenotype) (Tiemessen MM, et al. Proc Natl Acad Sci U S A. 2007 Dec 4;104(49):19446-51), raising the potential that these changes are a direct result of the increased number or functional capacity of Treg induced by ld IL-2 therapy. However, cells of the monocyte-macrophage lineage express functional IL-2 receptors (Ohashi Y, et al. J Immunol. 1989 Dec 1;143(11):3548-55; Wahl SM, et al. J Immunol. 1987 Aug 15;139(4):1342-7), and expression of CD25 (IL2RA) is increased under inflammatory conditions (Espinoza-Delgado I, et al. J Immunol. 1992 Nov 1;149(9):2961-8; Dendrou CA, et al. Nat Genet. 2009 Sep;41(9):1011-5), raising the possibility that macrophage/microglial polarisation may also occur as a direct result of ld IL-2 acting directly on these cells.

Finally, we could not observe statistically significant changes in plasma neurofilament light chain levels (NFL) according to treatment. Based on previously published data (Gaiottino J, et al. PloS One 2013; 8: e75091), we had estimated that it should be possible to detect a treatment effect with relatively few patients per group. However, in the context of this randomised (and strictly blinded) study, post-hoc power analysis suggests that this is a large underestimate, and that a much larger sample would have been needed in order to demonstrate the observed change in NFL plasma levels, notably in view of the large variability of NFL plasma levels in all treatment arms at baseline (Table 6 above). However, despite lack of statistical significance, there is a clear trend suggesting that while the placebo group is increasing plasma NFL overtime, the treated groups did not, which is consistent with a decrease in disease activity in relation to ld IL2 treatment.

In conclusion, this study shows that ld-IL-2 is safe over 3 monthly cycles in ALS subjects. In addition, we provide clear evidence for *in vivo* amplification of Treg numbers, frequency and function with ld IL-2. Since Treg function has been correlated to disease progression (Beers DR et al. JCI Insight. 2017;2(5):e89530; Thonhoff JR et al. Curr Opin Neurol 2018; 31: 635-9), these results support therapeutic efficiency of ld IL-2 in ALS patients. Importantly, in the light of the previous findings of raised CCL2 in plasma and CSF of ALS patients and correlation to disease score (Nagata T, et al. Neurol Res. 2007 Dec;29(8):772-6) and survival (Gille B et al. J Neurol Neurosurg Psychiatry. 2019 Dec;90(12):1338-1346), our observation that plasma CCL2 is decreased in dose-related fashion to ld IL-2 treatment further supports therapeutic efficiency of ld IL-2 in ALS patients. As a result, a phase 2/3 study based on these observations is ongoing (MIROCALS, ClinicalTrials.gov NCT03039673, see Example 2).

### Example 2: MIROCALS: Modifying Immune Response and OutComes in ALS (MIROCALS)

Following the very positive data obtained in the IMODALS trial, in particular concerning the significant drop in plasma concentration of CCL2, a marker of disease progression, and the change in polarization of macrophages from an inflammatory M1 phenotype towards an anti-inflammatory and repairing phenotype M2, a new phase II trial referred to as "MIROCALS" has been launched to confirm the positive effect of low dose human IL-2 on ALS.

### Patients and Methods

The "Modifying Immune Response and OutComes in ALS" project (MIROCALS) tests in a proof-of concept / proof of mechanism (PoC/ PoM) study the hypothesis that ld IL-2-treatment results in decreased rate of neuronal damage in ALS patients, as measured by early change in CSF neurofilament and CCL2 levels, and that this early change is predictive of long-term clinical efficacy as assessed by survival over an 18 months treatment period. ***Design:*** This is a double-blind, randomized, stratified (on country and site of onset) placebo-controlled, parallel group, study of low dose IL-2 at 2 MIU/day for 5 days every 4 weeks during 18 months. The study is preceded by a three-month run-in period to establish safety and stability of the riluzole treatment.

**Treatments:** All treatment packages consisting in 5 1ml prefilled polypropylene syringes containing either 0.6 ml of a 2MIU aldesleukine solution or 0.6 ml of 5% glucose water for injection are prepared, labelled and packaged in a central pharmacy in aseptic and temperature controlled conditions. In case of poor tolerance PIs are allowed to prescribe flexible dose down 1MIU (0.3ml) or 0.5MIU (0.15ml) to control for patient compliance. ***Primary end-points:*** Long-term survival over an 18-month treatment period, early changes (four months treatment) in CSF-pNFH and CCL2 levels.

**Secondary end-points:** Long-term safety of ld IL-2 therapy over 18 months of treatment, efficacy on functional decline (ALSFRS, Vital Capacity), chemokine marker for immuno-inflammation (CSF and blood), immuno-cytometry (blood)), and pNFH levels (CSF and blood).

***Ancillary studies:*** New biomolecular targets for drug intervention through genomics and transcriptomics profiling of ld IL-2 responder and non-responder patients.

***Measurements:*** Core laboratory measures (pNFH and CCL2 in CSF and blood, and blood immunocytometry) are performed in a central lab under GCLP conditions. Additional supportive immuno-inflammatory markers and omics investigations are performed in Academic laboratories.

***Patients inclusion criteria:** Run-in period selection:* De novo patients, Probable, or Laboratory-Supported Probable, or Definite ALS by El Escorial Revised ALS diagnostic criteria, disease duration ≤ 24 months, a vital capacity ≥ 70% of normal, no prior or present riluzole treatment, signed informed consent.

*RCT period:* same criteria except, disease duration ≤ 27 months, "no prior or present riluzole treatment" replaced by "stable on riluzole treatment for 3 months".

***Patient exclusion criteria:*** Contra indication to lumbar puncture; other life-threatening disease; other disease precluding functional assessments; cancer within the past 5 years (except stable non-metastatic basal cell skin carcinoma or in situ carcinoma of the cervix); severe cardiac or pulmonary disease; documented auto-immune disorders except asymptomatic Hashimoto thyroiditis, women of child bearing age without contraception or pregnant or breast feeding; any clinically significant laboratory abnormality (exception of cholesterol, triglyceride and glucose).

***Statistical analyses:*** The primary efficacy analysis compares the treatment groups on survival using (i) a stratified log rank test, and (ii) following adjustment on prognostic factor candidates (Cox model analysis), including age, vital capacity, & ALSFRS scores. Change in pNFH and CCL2 from randomisation to M4, and immunocytometry parameters are to be analysed using variance/ covariance analyses (ANOVA/ ANCOVA). Analysis of repeated measures of function (ALSFRS and SVC) is performed with joint rank analysis methods for informative censored data.

***Number of patients:*** Overall 216 patients are to be randomised with 108 patients per group to attain a power >0.80, at pα_{(2-sided)}= 0.05, to detect at 18 months an absolute difference in death rate of 17 % (placebo expected survival: 0.65 vs 0.82 in ld IL-2 group) - representing a 54% decrease in risk of death (RR ld IL-2/ PLA = 0.46). Given an attrition rate of 10% from eligible at selection to randomisation, about 240 patients are to be screened.

### Expected patient or public health benefit.

Based on the results obtained in IMODALS, in particular the ldIL2 positive impact in plasma concentrations of markers of disease activity (ie, decrease of CCL2 and NFL), and the change in immune-inflammatory parameters including improved Tregs suppressive function and shift of macrophages polarization from an inflammatory M1 phenotype towards an anti-inflammatory and repairing phenotype M2, a significant improvement of survival and decrease in rate of functional decline of ALS patients is expected from a treatment comprising repeated cycles of low dose human IL-2 subcutaneous injections. Due to poor knowledge of ALS pathogenesis and lack of predictive preclinical models, drug trials in ALS are pursuing two objectives, (i) testing clinical efficacy, and (ii) testing the pathogenic hypothesis justifying drug assessment. However, in "standard" trials, there is no means to know whether drug failure is due to a wrong hypothesis, or poor drug engagement of targeted pathways, or ineffective doses. By including in the design biomarkers quantifying both pharmacodynamics and disease activity responses to the drug, and by testing their surrogacy on the hard clinical end-point survival, a high level of evidence on ld IL-2 clinical efficacy, adding supporting PoC/PoM evidence is to be attained. Together with high GCPs/GCLPs quality settings, this study should therefore provide optimal Regulatory acceptance of the clinical results. In addition, this design will determine the strength of evidence of the pathogenic hypothesis being tested, while omics approaches will provide deep understanding of the drug effects, pathways involved, and their interactions with disease processes, eventually paving the way for new drug developments and further therapeutic progress.

## Claims

1. Human interleukin-2 (IL-2) for use in the treatment of amyotrophic lateral sclerosis in a human subject, wherein each dose of human IL-2 administered to said subject is between 0.1 x10⁶ to 3x10⁶ international units (IU) and said treatment does not comprise the administration of regulatory T-cells (Tregs) to the subject and does not comprise the administration of rapamycin or any other suppressive agent of effector T cells (Teffs) to the subject,
wherein the human IL-2 administered to the patient is not complexed with anti-human IL-2 antibodies.

2. Human IL-2 for use according to claim 1, wherein human IL-2 is administered as repeated injections of 0.1 x10⁶ to 3x10⁶ IU of human IL-2.

3. Human IL-2 for use according to claim 2, wherein repeated cycles of 3 to 7 consecutive days of once-daily sub-cutaneous injection of 0.1x10⁶ to 3x10⁶ IU human IL-2 are administered to the subject, preferably each cycle consists of 5 consecutive days of once-daily sub-cutaneous injection of 0.1 x10⁶ to 3 x10⁶ IU IL-2, more preferably 1 x10⁶ to 3 x10⁶ IU IL-2, most preferably 2x10⁶ IU human IL-2.

4. Human IL-2 for use according to claim 3, wherein the cycles are administered every 2 to 6 weeks, preferably every 2 to 5 weeks, more preferably every 2 to 4 weeks, in particular every 2, 3 or 4 weeks.

5. Human IL-2 for use according to claim 2, wherein IL2 is administered continuously according to a metronomic schedule.

6. Human IL-2 for use according to any one of claims 1 to 5, wherein said treatment is administered for the life-time of the subject.

7. Human IL-2 for use according to any one of claims 1 to 6, wherein human IL-2 is administered by subcutaneous or intravenous route, preferably by subcutaneous route.

8. Human IL-2 for use according to any one of claims 1 to 7, wherein said treatment further comprises administering riluzole to said subject.

9. Human IL-2 for use according to claim 8, wherein riluzole is orally administered at a daily dose of 50 mg to 100 mg, taken in two equal doses of 25 mg to 50 mg separated by about 12 hours.

10. Human IL-2 for use according to any one of claims 1 to 9, wherein said human IL-2 is a recombinant form of human IL-2.

11. Human IL-2 for use according to claim 10, wherein said human IL-2 is aldesleukin.

## Patentansprüche

1. Humanes Interleukin-2 (IL-2) zur Verwendung in der Behandlung von Amyotropher Lateralsklerose in einem menschlichen Subjekt, wobei jede an das Subjekt verabreichte Dosis humanes IL-2 zwischen 0,1 x 10⁶ bis 3 x 10⁶ internationale Einheiten (IU) ist und die Behandlung nicht die Verabreichung von regulatorischen T-Zellen (Tregs) an das Subjekt umfasst und nicht die Verabreichung von Rapamycin oder eines anderen suppressiven Agens von Effektor-T-Zellen (Teffs) an das Subjekt umfasst,
wobei das an den Patienten verabreichte humane IL-2 nicht mit Anti-humanen IL-2 Antikörpern komplexiert ist.

2. Humanes IL-2 zur Verwendung nach Anspruch 1, wobei das humane IL-2 als wiederholte Injektionen von 0,1 x. 10⁶ bis 3 x 10⁶ IU des humanen IL-2 verabreicht wird.

3. Humanes IL-2 zur Verwendung nach Anspruch 2, wobei wiederholte Zyklen von 3 bis 7 aufeinanderfolgenden Tagen von einmal täglich subkutaner Injektion von 0,1 x 10⁶ bis 3 x 10⁶ IU humanes IL-2 an das Subjekt verabreicht werden, bevorzugt besteht jeder Zyklus aus 5 aufeinanderfolgenden Tagen einmal täglicher subkutaner Injektion von 0,1 x 10⁶ bis 3 x 10⁶ IU IL-2, mehr bevorzugt 1 x 10⁶ bis 3 x 10⁶ IU IL-2,am meisten bevorzugt 2 x 10⁶ IU humanes IL-2.

4. Humanes IL-2 zur Verwendung nach Anspruch 3, wobei die Zyklen alle 2 bis 6 Wochen verabreicht werden, bevorzugt alle 2 bis5 Wochen, mehr bevorzugt alle 2 bis 4 Wochen, insbesondere alle 2, 3 oder 4 Wochen.

5. Humanes IL-2 zur Verwendung nach Anspruch 2, wobei IL-2 kontinuierlich nach einem metronomischen Schema verabreicht wird.

6. Humanes IL-2 zur Verwendung nach einem der Ansprüche 1 bis 5, wobei die Behandlung für die Lebenszeit des Subjekts verabreicht wird.

7. Humanes IL-2 zur Verwendung nach einem der Ansprüche 1 bis 6, wobei humanes IL-2 über den subkutanen oder intravenösen Weg, bevorzugt über den subkutanen Weg verabreicht wird.

8. Humanes IL-2 zur Verwendung nach einem der Ansprüche 1 bis 7, wobei die Behandlung ferner die Verabreichung von Riluzol an das Subjekt umfasst.

9. Humanes IL-2 zur Verwendung nach Anspruch 8, wobei Riluzol oral in einer täglichen Dosis von 50 mg bis 100 mg, die in zwei gleichen Dosen von 25 mg bis 50 mg im Abstand von 12 Stunden genommen wird, verabreicht wird.

10. Humanes IL-2 zur Verwendung nach einem der Ansprüche 1 bis 9, wobei das humane IL-2 eine rekombinante Form des humanen IL-2 ist.

11. Humanes IL-2 zur Verwendung nach Anspruch 10, wobei das humane IL-2 Aldesleukin ist.

## Revendications

1. Interleukine 2 (IL-2) humaine pour une utilisation dans le traitement de la sclérose latérale amyotrophique chez un sujet humain, dans laquelle chaque dose d'IL-2 humaine administrée audit sujet est comprise entre 0,1 x 10⁶ et 3 x 10⁶ unités internationales (UI) et ledit traitement ne comprend pas l'administration de cellules T régulatrices (Treg) au sujet et ne comprend pas l'administration de rapamycine ou d'un quelconque autre agent suppresseur de cellules T effectrices (Teff) au sujet,
dans laquelle l'IL-2 humaine administrée au patient n'est pas complexée avec des anticorps anti-IL-2 humaine.

2. IL-2 humaine pour une utilisation selon la revendication 1, laquelle IL-2 humaine est administrée sous la forme d'injections répétées de 0,1 x 10⁶ à 3 x 10⁶ UI d'IL-2 humaine.

3. IL-2 humaine pour une utilisation selon la revendication 2, dans laquelle des cycles répétés de 3 à 7 jours consécutifs d'injection sous-cutanée une fois par jour de 0,1 x 10⁶ à 3 x 10⁶ UI d'IL-2 humaine sont administrés au sujet, de préférence chaque cycle consiste en 5 jours consécutifs d'injection sous-cutanée une fois par jour de 0,1 x 10⁶ à 3 x 10⁶ UI d'IL-2 humaine, mieux encore de 1 x 10⁶ à 3 x 10⁶ UI d'IL-2, tout spécialement de 2 x 10⁶ UI d'IL-2 humaine.

4. IL-2 humaine pour une utilisation selon la revendication 3, dans laquelle les cycles sont administrés toutes les 2 à 6 semaines, de préférence toutes les 2 à 5 semaines, mieux encore toutes les 2 à 4 semaines, en particulier toutes les 2, 3 ou 4 semaines.

5. IL-2 humaine pour une utilisation selon la revendication 2, laquelle IL-2 est administrée en continu conformément à un schéma métronomique.

6. IL-2 humaine pour une utilisation selon l'une quelconque des revendications 1 à 5, dans laquelle ledit traitement est administré pendant toute la vie du sujet.

7. IL-2 humaine pour une utilisation selon l'une quelconque des revendications 1 à 6, laquelle IL-2 humaine est administrée par voie sous-cutanée ou intraveineuse, de préférence par voie sous-cutanée.

8. IL-2 humaine pour une utilisation selon l'une quelconque des revendications 1 à 7, dans laquelle ledit traitement comprend en outre l'administration de riluzole audit sujet.

9. IL-2 humaine pour une utilisation selon la revendication 8, dans laquelle le riluzole est administré par voie orale à une dose quotidienne de 50 mg à 100 mg, prise en deux doses identiques de 25 mg à 50 mg séparées par environ 12 heures.

10. IL-2 humaine pour une utilisation selon l'une quelconque des revendications 1 à 9, laquelle IL-2 humaine est une forme recombinée d'IL-2 humaine.

11. IL-2 humaine pour une utilisation selon la revendication 10, laquelle IL-2 humaine est l'aldesleukine.
